(19) Europäisches Patentamt / European Patent Office / Office européen des brevets

(11) **EP 4 435 107 A1**

(12) **EUROPEAN PATENT APPLICATION**
published in accordance with Art. 153(4) EPC


(43) Date of publication:
**25.09.2024 Bulletin 2024/39**

(21) Application number: **22898449.8**

(22) Date of filing: **14.11.2022**

(51) International Patent Classification (IPC):
***C12N 15/12*** *(2006.01)* ***B01J 20/281*** *(2006.01)*
***C07K 1/22*** *(2006.01)* ***C07K 14/735*** *(2006.01)*
***C07K 16/00*** *(2006.01)* ***C07K 17/02*** *(2006.01)*
***G01N 30/26*** *(2006.01)* ***G01N 30/34*** *(2006.01)*
***G01N 30/88*** *(2006.01)*

(52) Cooperative Patent Classification (CPC):
**B01J 20/281; C07K 1/22; C07K 14/435; C07K 14/70535; C07K 16/00; C07K 17/02; G01N 30/26; G01N 30/34; G01N 30/88**

(86) International application number:
**PCT/JP2022/042273**

(87) International publication number:
**WO 2023/095665 (01.06.2023 Gazette 2023/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2021 JP 2021192859**
**14.02.2022 JP 2022020578**
**24.08.2022 JP 2022133470**



(71) Applicant: **Tosoh Corporation**
**Yamaguchi 746-8501 (JP)**

(72) Inventors:
- **HAYAKAWA Yuta**
**Ayase-shi, Kanagawa 252-1123 (JP)**
- **TANIGUCHI Naomasa**
**Ayase-shi, Kanagawa 252-1123 (JP)**
- **TERAO Yosuke**
**Ayase-shi, Kanagawa 252-1123 (JP)**

(74) Representative: **Novagraaf International SA**
**Chemin de l'Echo 3**
**1213 Onex, Geneva (CH)**


Remarks:
The complete document including Reference Table(s) and the Sequence Listing(s) can be downloaded from the EPO website

(54) **ANTIBODY ISOLATION METHOD**


(57) An antibody separation method including a step of applying a solution containing an antibody to a column filled with an antibody adsorption material containing an insoluble porous carrier and an Fc-binding protein immobilized on the carrier and adsorbing the antibody onto the antibody adsorption material; and a step of applying a buffer solution to the column and eluting the antibody adsorbed onto the antibody adsorption material with a pH gradient, wherein the pH gradient is a step gradient in which a pH of the buffer solution is lowered in steps, and wherein the magnitude of pH decrease is 0.1 or more and less than 1.


***Fig.6***

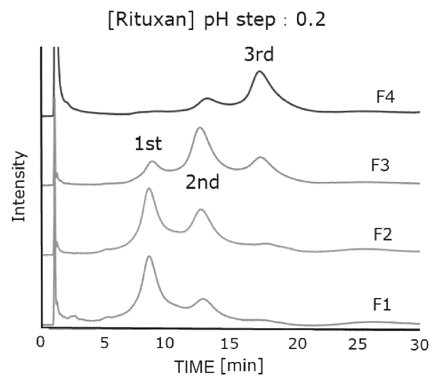


EP 4 435 107 A1

## Description

### Technical Field

The present invention relates to an antibody separation method using an antibody adsorption material. More specifically, the present invention relates to an antibody adsorption material in which a step of eluting an antibody adsorbed onto the antibody adsorption material is optimized.

### Background Art

Antibody drugs are drugs that use antibodies (immunoglobulins), which are molecules having an immune function in living organisms. The antibody drugs bind to target molecules with high specificity and affinity due to the diversity of variable regions of the antibodies. Therefore, the antibody drugs have fewer side effects, and in recent years, the number of diseases for which the antibody drugs can be applied has been increasing, and the market has been rapidly expanding.

Antibodies used in antibody drugs are produced by culturing cells (for example, Chinese hamster ovary cells, etc.) that can express the antibodies obtained by a genetic engineering method and then purifying them to a high purity using column chromatography or the like.

In recent years, it has been found that antibody drugs undergo modifications such as oxidation, reduction, isomerization or glycosylation, resulting in aggregates of diverse molecules, and there are concerns about the influence on drug efficacy and safety. Particularly, it has been reported that a difference in structure of sugar chain bound to an antibody has a significant influence on the activity, kinetics and safety of antibody drugs (Non-Patent Literature 1), and during production, it is required to separate the structure of sugar chain, that is, each adsorption activity to the chromatography carrier.

Among separations using chromatography, in separation using affinity chromatography, since separation is performed based on the affinity between an affinity ligand and an antibody, it may be possible to perform separation based on minute structural changes that depend on the adsorption activity to chromatography carriers. For example, according to an affinity chromatography method using an antibody adsorption material containing an insoluble porous carrier and an Fc-binding protein immobilized on the carrier, an antibody can be separated and fractionated based on a difference in structure of sugar chain (for example, a difference in antibody-dependent cellular cytotoxicity (ADCC) activity) (Patent Literature 1).

In the affinity chromatography method disclosed in Patent Literature 1, an antibody adsorbed onto an antibody adsorption material is eluted using a linear gradient in which the pH is continuously lowered. However, when elution according to a linear gradient is applied, separation of elution peaks deteriorates, much time is required to separate an antibody, and thus it is difficult to treat a large amount of antibodies.

### Citation List

#### Patent Literature

[Patent Literature 1] Japanese Unexamined Patent Publication No. 2018-197224

#### Non-Patent Literature

[Non-Patent Literature 1] Y. Yagi and S. Suzuki, "Characterization of Oligosaccharides in Therapeutic Antibodies," CHROMATOGRAPHY, 34(2), 83-88 (2013)

### Summary of Invention

#### Technical Problem

An object of the present invention is to provide a method with which a large amount of antibodies can be separated based on a difference in antibody-dependent cellular cytotoxicity activity due to a difference in structure of sugar chain in an affinity chromatography method using an antibody adsorption material containing an insoluble porous carrier and an Fc-binding protein immobilized on the carrier.

## Solution to Problem

In order to achieve the above object, the inventors conducted extensive studies, and as a result, found that, when antibody adsorbed onto the antibody adsorption material is eluted with a step gradient in which the pH is lowered in steps, and the magnitude of decrease is optimized, the present invention is achieved.

Specifically, the present invention includes the following aspects [1] to [24].

[1] A method for separating an antibody including a step of applying a solution containing an antibody to a column filled with an antibody adsorption material containing an insoluble porous carrier and an Fc-binding protein immobilized on the carrier and adsorbing the antibody onto the antibody adsorption material; and a step of applying a buffer solution to the column and eluting the antibody adsorbed onto the antibody adsorption material with a pH gradient, wherein the pH gradient is a step gradient in which a pH of the buffer solution is lowered in steps, and wherein the magnitude of pH decrease is 0.1 or more and less than 1.

[2] The method according to [1], wherein the insoluble porous carrier has a volume-based Mean particle Diameter of 30 to 150 μm.

[3] The method according to [1] or [2], wherein the buffer solution is a solution containing a buffer substance that has a buffering capacity in a pH range of 3.0 or more and 6.0 or less and has a plurality of pKas within the range.

[4] The method according to any one of [1] to [3], wherein a ratio of a mass of the antibody to the volume of the antibody adsorption material is 1 mg/1.0 mLto 50 mg/0.1 mL.

[5] The method according to any one of [1] to [4], wherein the Fc-binding protein is human FcyRIIIa.

[6] The method according to [5], wherein the human FcyRIIIa is a polypeptide shown in the following (1), (2) or (3):

(1) a polypeptide including the amino acid sequence from the 17th glycine residue (Gly) to the 192nd glutamine residue (Gln) in the amino acid sequence set forth as SEQ ID NO: 1,

(2) a polypeptide having one or more amino acid residue substitutions, deletions, insertions or additions in the amino acid sequence from the 17th glycine residue (Gly) to the 192nd glutamine residue (Gln) in the amino acid sequence set forth as SEQ ID NO: 1, and having binding activity to the antibody, and

(3) a polypeptide having a homology of 70% or more with the amino acid sequence from the 17th glycine residue (Gly) to the 192nd glutamine residue (Gln) in the amino acid sequence set forth as SEQ ID NO: 1 and having binding activity to the antibody.

[7] An antibody composition containing 15 mass% or more of an antibody which is eluted in the following citrate buffer solution with a pH of 4.0 or less when a monoclonal antibody solution is fractionated under the following measurement conditions, based on a total mass of the antibody composition.

[8] An antibody composition containing 15 mass% or more of an antibody which is detected with a retention time of 750 minutes or longer when a monoclonal antibody solution is fractionated under the following measurement conditions, based on a total mass of the antibody composition.

[9] An antibody composition containing 15 mass% or more of an antibody which is eluted in the following citrate buffer solution with a pH of 4.2 or more when a monoclonal antibody solution is fractionated under the following measurement conditions, based on a total mass of the antibody composition.

[10] An antibody composition containing 15 mass% or more of an antibody which is detected with a retention time of shorter than 750 minutes when a monoclonal antibody solution is fractionated under the following measurement conditions, based on a total mass of the antibody composition.

[11] A concentrate of an antibody solution, wherein the antibody solution and the concentrate contain the antibody which is eluted in the following citrate buffer solution with a pH of 4.0 or less when a monoclonal antibody solution is fractionated under the following measurement conditions, and wherein a content of the antibody based on a total amount of the concentrate is at least twice a content of the antibody based on a total amount of the antibody solution.

[12] A concentrate of an antibody solution, wherein the antibody solution and the concentrate contain the antibody which is detected with a retention time of 750 minutes or longer when a monoclonal antibody solution is fractionated under the following measurement conditions, and wherein a content of the antibody based on a total amount of the concentrate is at least twice a content of the antibody based on a total amount of the antibody solution.

[13] A concentrate of an antibody solution, wherein the antibody solution and the concentrate contain the antibody which is eluted in the following citrate buffer solution with a pH of 4.2 or more when a monoclonal antibody solution is fractionated under the following measurement conditions, and wherein a content of the antibody based on a total amount of the concentrate is at least twice acontent of the antibody based on a total amount of the antibody solution.

[14] A concentrate of an antibody solution, wherein the antibody solution and the concentrate contain the antibody which is detected with a retention time of shorter than 750 minutes when a monoclonal antibody solution is fractionated under the following measurement conditions, and wherein a content of the antibody based on a total amount of the

concentrate is at least twice a content of the antibody based on a total amount of the antibody solution.

(Measurement conditions) in [7] to [14]:

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.07 mL/min
Solvent: phosphate buffered saline (pH 7.4)
Step gradient: add 3.73 mL each of a 50 mM citrate buffer solution (pH 6.0), a 50 mM citrate buffer solution (pH 5.5), a 50 mM citrate buffer solution (pH 5.0), a 50 mM citrate buffer solution (pH 4.8), a 50 mM citrate buffer solution (pH 4.6), a 50 mM citrate buffer solution (pH 4.4), a 50 mM citrate buffer solution (pH 4.2), a 50 mM citrate buffer solution (pH 4.0), a 50 mM citrate buffer solution (pH 3.8), a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)
Injection amount: 2,000 μL (antibody amount: 5 mg)

An antibody composition containing 15 mass% or more of an antibody which is eluted in the following citrate buffer solution with a pH of 4.0 or less when a monoclonal antibody solution is fractionated under the following measurement conditions, based on a total mass of the antibody composition.

An antibody composition containing 15 mass% or more of an antibody which is detected with a retention time of 800 minutes or longer when a monoclonal antibody solution is fractionated under the following measurement conditions, based on a total mass of the antibody composition.

An antibody composition containing 15 mass% or more of an antibody which is eluted in the following citrate buffer solution with a pH of 4.2 or more when a monoclonal antibody solution is fractionated under the following measurement conditions, based on a total mass of the antibody composition.

An antibody composition containing 15 mass% or more of an antibody which is detected with a retention time of shorter than 800 minutes when a monoclonal antibody solution is fractionated under the following measurement conditions, based on a total mass of the antibody composition.

A concentrate of an antibody solution, wherein the antibody solution and the concentrate contain the antibody which is eluted in the following citrate buffer solution with a pH of 4.0 or less when a monoclonal antibody solution is fractionated under the following measurement conditions, and wherein a content of the antibody based on a total amount of the concentrate is at least twice a content of the antibody based on a total amount of the antibody solution.

A concentrate of an antibody solution, wherein the antibody solution and the concentrate contain the antibody which is detected with a retention time of 800 minutes or longer when a monoclonal antibody solution is fractionated under the following measurement conditions, and wherein a content of the antibody based on a total amount of the concentrate is at least twice a content of the antibody based on a total amount of the antibody solution.

A concentrate of an antibody solution, wherein the antibody solution and the concentrate contain the antibody which is eluted in the following citrate buffer solution with a pH of 4.2 or more when a monoclonal antibody solution is fractionated under the following measurement conditions, and wherein a content of the antibody based on a total amount of the concentrate is at least twice a content of the antibody based on a total amount of the antibody solution.

A concentrate of an antibody solution, wherein the antibody solution and the concentrate contain the antibody which is detected with a retention time of shorter than 800 minutes when a monoclonal antibody solution is fractionated under the following measurement conditions, and wherein a content of the antibody based on a total amount of the concentrate is at least twice a content of the antibody based on a total amount of the antibody solution.

(Measurement conditions) in [15] to [22]:

Column: Tricorn 5/50 (φ5.0 mm×50 mm, commercially available from Cytiva)

Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.20 mL/min
Solvent: phosphate buffered saline (pH 7.4)
Step gradient: add 9.82 mL each of a 50 mM citrate buffer solution (pH 6.0), a 50 mM citrate buffer solution (pH 5.5), a 50 mM citrate buffer solution (pH 5.0), and a 50 mM citrate buffer solution (pH 4.8) in that order, add 29.46 mL each of a 50 mM citrate buffer solution (pH 4.6), a 50 mM citrate buffer solution (pH 4.4), a 50 mM citrate buffer solution (pH 4.2), a 50 mM citrate buffer solution (pH 4.0), and a 50 mM citrate buffer solution (pH 3.8) in that order, and add 9.82 mL each of a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)
Injection amount: 1000-8000 μL (antibody amount: 5-40 mg)

The method according to any one of [1] to [6], wherein the method is based on a difference in a structure of sugar chain of the antibody.
The method according to [23], wherein the difference in the structure of sugar chain is a difference in the number of galactose residues at the non-reducing end in the structure of sugar chain.

## Advantageous Effects of Invention

In the present invention, when an antibody contained in a solution is separated using an antibody adsorption material containing an insoluble porous carrier and an Fc-binding protein immobilized on the carrier, an antibody adsorbed onto the adsorption material is eluted with a step gradient in which the pH is lowered in steps in a magnitude of decrease of 0.1 or more and less than 1. According to the present disclosure, it is easier to separate an antibody having a desired structure (for example, a structure of sugar chain) and activity (for example, ADCC activity or complement-dependent cytotoxicity (CDC) activity) and it is possible to reduce lot-to-lot differences in separated antibody, compared to elution using a linear gradient in which the pH is continuously lowered, which has been conventionally performed. In addition, since the insoluble porous carrier of an embodiment of the present invention has a large particle size and is porous, it is possible to further improve the dynamic binding capacity (DBC) and reduce pressure loss. As described above, the present disclosure is useful as a separation method for producing a large amount of antibodies, which is used in industrial production of antibody drugs.

## Brief Description of Drawings

FIG. 1 is a diagram showing a pH approximate curve of a citrate buffer solution.
FIG. 2 is a diagram showing step gradients implemented in Examples 1 to 6 and Comparative Example 1.
FIG. 3 is a diagram showing separation patterns of an FcR36i_Cys column in Example 1 (Rituxan, a pH decrease by 0.5).
FIG. 4 is a diagram showing the results (separation patterns) obtained by subjecting an F1 fraction and an F2 fraction in FIG. 3 to an FcRIIIA column.
FIG. 5 is a diagram showing separation patterns of an FcR36i_Cys column in Example 2 (Rituxan, a pH decrease by 0.2).
FIG. 6 is a diagram showing the results (separation patterns) obtained by subjecting an F1 fraction to an F4 fraction in FIG. 5 to an FcRIIIA column.
FIG. 7 is a diagram showing separation patterns of an FcR36i_Cys column in Comparative Example 1 (Rituxan, a pH decrease by 1.0).
FIG. 8 is a diagram showing the results (separation patterns) obtained by subjecting an F1 fraction in FIG. 7 to an FcRIIIA column.
FIG. 9 is a diagram showing separation patterns of an FcR36i_Cys column in Example 3 (Actemra, a pH decrease by 0.5).
FIG. 10 is a diagram showing the results (separation patterns) obtained by subjecting an F1 fraction to an F3 fraction

in FIG. 9 to an FcRIIIA column.

FIG. 11 is a diagram showing separation patterns of an FcR36i_Cys column in Example 4 (Actemra, a pH decrease by 0.2).

FIG. 12 is a diagram showing the results (separation patterns) obtained by subjecting an F1 fraction to an F6 fraction in FIG. 11 to an FcRIIIA column.

FIG. 13 is a diagram showing separation patterns of an FcR36i_Cys column in Example 5 (Erbitux, a pH decrease by 0.5).

FIG. 14 is a diagram showing the results (separation patterns) obtained by subjecting an F1 fraction and an F2 fraction in FIG. 13 to an FcRIIIA column.

FIG. 15 is a diagram showing separation patterns of an FcR36i_Cys column in Example 6 (Erbitux, a pH decrease by 0.2).

FIG. 16 is a diagram showing the results (separation patterns) obtained by subjecting an F1 fraction to an F4 fraction in FIG. 15 to an FcRIIIA column.

FIG. 17 is a diagram showing a step gradient implemented in Examples 7 to 11.

FIG. 18 is a diagram showing separation patterns of an FcR36i_Cys column in Example 7 (Rituxan, a pH decrease by 0.2).

FIG. 19 is a diagram showing the results (separation patterns) obtained by subjecting an F1 fraction to an F3 fraction in FIG. 18 to an FcRIIIA column.

FIG. 20 is a diagram showing separation patterns of an FcR36i_Cys column in Example 8 (Rituxan, a pH decrease by 0.2).

FIG. 21 is a diagram showing the results (separation patterns) obtained by subjecting an F1 fraction to an F4 fraction in FIG. 20 to an FcRIIIA column.

FIG. 22 is a diagram showing separation patterns of an FcR36i_Cys column in Example 9 (Rituxan, a pH decrease by 0.2).

FIG. 23 is a diagram showing the results (separation patterns) obtained by subjecting an F1 fraction to an F4 fraction in FIG. 22 to an FcRIIIA column.

FIG. 24 is a diagram showing separation patterns of an FcR36i_Cys column in Example 10 (Rituxan, a pH decrease by 0.2).

FIG. 25 is a diagram showing the results (separation patterns) obtained by subjecting an F1 fraction to an F6 fraction in FIG. 24 to an FcRIIIA column.

FIG. 26 is a diagram showing separation patterns of an FcR36i_Cys column in Example 11 (Rituxan, a pH decrease by 0.2).

FIG. 27 is a diagram showing the results (separation patterns) obtained by subjecting an F1 fraction to an F6 fraction in FIG. 26 to an FcRIIIA column.

FIG. 28 is a diagram showing the proportions of structures of sugar chain of an antibody contained in the F2 fraction in FIG. 18 and the F1, F2 and F4 fractions in FIG. 20.

**Description of Embodiments**

Hereinafter, embodiments (hereinafter simply referred to as "the present embodiment") for implementing the present invention will be described in detail. The present embodiment below is an example for explaining the present invention, and there is no intention to limit the present invention to the following content. The present invention can be implemented with appropriate modifications within the scope of the spirit of the present invention.

A method for separating an antibody according to the present invention includes a step of applying a solution containing an antibody to a column filled with an antibody adsorption material containing an insoluble porous carrier and an Fc-binding protein immobilized on the carrier and adsorbing the antibody onto the antibody adsorption material, and a step of applying a buffer solution to the column and eluting the antibody adsorbed onto the antibody adsorption material with a pH gradient, the pH gradient is a step gradient in which the pH of the buffer solution is lowered in steps, and the magnitude of pH decrease is 0.1 or more and less than 1.

The insoluble porous carrier in the present invention may be any substance that can immobilize Fc-binding proteins and is insoluble in a solution or solvent used for antibody adsorption/elution. The insoluble porous carrier may be a carrier derived from an inorganic substance such as zirconia, zeolite, silica, or coated silica, a carrier derived from a natural organic polymer substance such as cellulose, agarose, or dextran, or a carrier derived from a synthetic organic polymer substance such as a polyacrylic acid, polystyrene, polyacrylamide, polymethacrylamide, polymethacrylate, or vinyl polymer. In addition, commercially available chromatography carriers such as Shodex (commercially available from Showa Denko K.K.), Sepharose (commercially available from Cytiva), Amberlite (commercially available from Du Pont Inc.), Cellufine (commercially available from JNC), POROS (commercially available from Thermo Fisher Scientific), and TOYOPEARL (commercially available from Tosoh Corporation) may be used as the insoluble porous carrier.

In the present invention, the insoluble porous carrier is a porous carrier. In this specification, the porous carrier is a sponge-like carrier that has pores on its surface and is hollow inside. More specifically, the porous carrier of an embodiment of the present invention may be a carrier with a porosity of 30% or more and 95% or less (preferably 50% or more and 95% or less).

The porosity can be calculated, for example, by the following steps (a) to (e), which are general methods in the field of liquid chromatography (PCT International Publication No. WO2017/141910).

(a) a step of filling a chromatography column with an insoluble porous carrier to be measured.
(b) a step of applying water to the column and measuring elution volumes of blue dextran (a molecular weight of 2,000,000) and sodium chloride from the column using water as an eluent.
(c) a step of calculating a gel volume from the difference between the column volume of the column and the elution volume of blue dextran measured in the above (b).
(d) a step of calculating a pore volume from the difference between the elution volume of sodium chloride measured in the operation (b) and the elution volume of molecular blue dextran measured in the operation (b).
(e) a step of calculating a porosity by dividing the pore volume calculated in the operation (d) by the gel volume calculated in the operation (c).

To summarize the above, the porosity can be calculated from the elution volume (Vn) of sodium chloride, the elution volume (Vo) of blue dextran (a molecular weight of 2,000,000) and the column volume (Vc) according to the following calculation formula.

$$\text{Porosity}[\%]=((\text{Vn}-\text{Vo})/(\text{Vc}-\text{Vo}))\times 100$$

The volume-based Mean particle Diameter of the insoluble porous carrier may be 30 to 150 μm. When the volume-based Mean particle Diameter is within this range, it is possible to further improve the dynamic binding capacity and to reduce the pressure loss. The volume-based Mean particle Diameter of the insoluble porous carrier can be determined by measuring the particle size distribution using a laser diffraction method.

In the present invention, the Fc-binding protein immobilized on an insoluble porous carrier is a protein having a property of binding to the Fc region of an antibody (an immunoglobulin). Examples of the Fc-binding protein include an Fc receptor, Protein A derived from Staphylococcus aureus, and Protein G derived from group G hemolytic streptococcus (group G streptococcus). In addition, specific examples of the Fc-binding protein being a human Fc receptor include human FcγRI, human FcγRIIa, human FcγRIIb, human FcγRIIIa, and human FcRn. Particularly, human FcγRIIIa is a human Fc receptor that can recognize a difference in a structure of sugar chain (for example, a structure contributing to antibody-dependent cellular cytotoxicity (ADCC) activity) of an antibody. An antibody adsorption material containing an insoluble porous carrier and human FcγRIIIa immobilized on the carrier can separate an antibody based on the difference in the structure of sugar chain of the antibody (for example, a difference in ADCC activity) (Japanese Unexamined Patent Publication No. 2018-197224). In some embodiments, the Fc-binding protein immobilized on an insoluble porous carrier is human FcγRIIIa. Examples of the difference in structure of sugar chain include the presence or absence of galactose residues at the non-reducing terminal and the difference in the number of galactose residues.

Examples of a preferable aspect of the Fc-binding protein in the present invention include

(1) a polypeptide including at least an extracellular region of natural human FcγRIIIa (the amino acid residues from the 17th glycine residue (Gly) to the 192nd glutamine residue (Gln) in the amino acid sequence set forth as SEQ ID NO: 1),
(2) a polypeptide including at least an extracellular region of natural human FcγRIIIa, and having one or more amino acid residue substitutions, deletions, insertions or additions in the amino acid residues constituting the extracellular region, and having antibody binding activity, and
(3) a polypeptide including at least an extracellular region of natural human FcγRIIIa, and having a homology of 70% or more with the amino acid sequence from the positions 17 to 192, and having binding activity to the antibody.

In the above (2), "one or more" means, for example, 1 or more and 40 or less, 1 or more and 30 or less, 1 or more and 20 or less, or 1 or more and 10 or less (10, 9, 8, 7, 6, 5, 4, 3, 2 or 1). Preferable aspects of the above (2) include an Fc-binding protein disclosed in Japanese Unexamined Patent Publication No. 2015-086216, an Fc-binding protein disclosed in Japanese Unexamined Patent Publication No. 2016-169197, an Fc-binding protein disclosed in Japanese Unexamined Patent Publication No. 2017-118871, an Fc-binding protein disclosed in Japanese Unexamined Patent Publication No. 2018-197224, and an Fc-binding protein disclosed in PCT International Publication No. WO2019/083048. When the Fc-binding protein is a protein shown in (2), alkali resistance may be improved.

The homology of the amino acid sequence in the above (3) may be 70% or more or may be higher (for example, 80% or more, 85% or more, 90% or more or 95% or more). When the Fc-binding protein is a protein shown in (3), alkali resistance may be improved.

In order to produce an antibody adsorption material used in an embodiment of the present invention by immobilizing an Fc-binding protein on the above insoluble porous carrier, for example, an activated functional group such as an N-hydroxysuccinimide (NHS) activated ester group, an epoxy group, a carboxy group, a maleimide group, a haloacetyl group, a tresyl group, a formyl group, or a haloacetamide (iodoacetamide, bromoacetamide, etc.) may be added to an insoluble porous carrier surfaces under appropriate reaction conditions, and the Fc-binding protein and the insoluble porous carrier may be then covalently bonded via the activated functional group for production. Alternatively, a commercially available chromatography carrier to which an activated functional group is added in advance may be covalently bonded to an Fc-binding protein for production. Examples of commercially available carriers to which an activated functional group is added include TOYOPEARL AF-Epoxy-650M and TOYOPEARL AF-Tresyl-650M (all commercially available from Tosoh Corporation), HiTrap NHS-activated HP Columns, NHS-activated Sepharose 4 Fast Flow, and Epoxy-activated Sepharose 6B (all commercially available from Cytiva), and SulfoLink Coupling Resin (commercially available from Thermo Fisher Scientific).

Here, when the insoluble porous carrier has a functional group such as a hydroxy group, an epoxy group, a carboxy group, or an amino group on its surface, a compound having an activated functional group is introduced into the functional group to produce an insoluble porous carrier to which the activated functional group is added, and an antibody adsorption material may be then produced by covalently bonding the activated functional group and an Fc-binding protein. For example, when the carrier has a hydroxy group on its surface, an activated functional group may be added using an activating agent such as epichlorohydrin (forms an epoxy group as an activating group), 1,4-butanediol diglycidyl ether (forms an epoxy group as an activating group), tresyl chloride (forms a tresyl group as an activating group), or vinyl bromide (forms a vinyl group as an activating group). In addition, after a hydroxy group is converted into an amino group or a carboxy group, an activated functional group may be added using an activating agent such as N-succinimidyl 3-maleimidopropionate (forms a maleimide group as an activating group), 1,1'-carbonyldiimidazole (forms a carbonylimidazole group as an activating group), or halogenated acetic acid (forms a halogenated acetyl group as an activating group).

The column filled with an antibody adsorption material may be one commonly used for affinity chromatography. The diameter (inner diameter) of the column can be 3 mm to 100 cm, and may preferably be 3 mm to 100 mm, and more preferably 3 mm to 30 mm. The length of the column can be 10 to 500 mm, and as the column, for example, a φ5.0 mm×50 mm Tricorn column (Tricorn 5/50, commercially available from Cytiva) can be used.

In the present invention, an antibody is a molecule containing an Fc region of immunoglobulin. The antibody may include an Fc region, or may include other regions in addition to the Fc region. The antibody of an embodiment of the present invention may have a sugar chain added thereto (have a sugar chain). The antibody may be a monoclonal antibody or a polyclonal antibody. The antibody may be an antibody drug. Examples of the antibody drug include Herceptin (trastuzumab), Rituxan (rituximab), Avastin (bevacizumab), Erbitux (cetuximab), Actemra (tocilizumab), and Remicade (infliximab). Commercially available antibody may be prepared, or an antibody may be produced using CHO cells (Chinese hamster ovary cell), Sp2/0 cells, NS0 cells, hybridoma cells or the like.

In an embodiment of the present invention, the solution containing an antibody may be one obtained by dissolving an antibody in a phosphate buffered saline.

In order to separate an antibody using the above antibody adsorption material, first, an equilibration solution may be applied to the column filled with the antibody adsorption material using a liquid transfer unit such as a pump, and thus the column may be equilibrated. Next, a solution containing an antibody may be added using the liquid transfer unit, and thus the antibody is specifically adsorbed onto the antibody adsorption material. Then, the adsorbed antibody may be eluted by applying a buffer solution using the liquid transfer unit and changing the pH stepwise. In the present invention, separation of an antibody is not limited to separation based on the structure, properties, activity and the like, but also includes separation of an antibody from a solution containing impurities (removal of impurities).

Examples of equilibration solutions include a phosphate buffer solution, an acetate buffer solution, an MES(2-(N-morpholino) ethanesulfonic acid) buffer solution, and a citrate buffer solution. An inorganic salt such as a 10 mM or more and 200 mM or less sodium chloride may be added to these buffer solutions. An optimal value of the pH of the equilibration solution may be appropriately determined from a pH range of 4.0 or more and 8.0 or less in consideration of the buffer solution component, the column shape, the pressure of the adsorption material filled into the column, and the like.

In the present invention, as described above, the antibody adsorbed onto the antibody adsorption material are eluted with a pH gradient. In the present invention, the pH gradient is a step gradient in which the pH of the buffer solution is lowered in steps, and the magnitude of pH decrease is 0.1 or more and less than 1.

A linear gradient may generally be used as the pH gradient, but when elution with a linear gradient is performed, if the slope of pH decrease is too steep, all antibodies may be eluted at once regardless of a difference in structure or activity. In addition, in response to the above issue, if the slope of pH decrease is made gentler, much time is required

and the obtained chromatogram (separation pattern) has gentle peaks, and thus it is difficult to separate an antibody based on the peaks. That is, it is difficult to determine a timing at which desired antibody is separated. Therefore, in the present invention, elution of the antibody adsorbed onto the antibody adsorption material with a pH gradient is performed with a step gradient.

In elution with a step gradient in the present invention, specifically, an antibody adsorbed onto the antibody adsorption material are eluted by lowering, in steps, the pH of the buffer solution applied to the column filled with the adsorption material by a magnitude of decrease of 0.1 or more and less than 1. When the magnitude of pH decrease is within this range, all antibodies are not eluted at once, and it becomes easy to separate an antibody based on peaks. As a result, the antibody can be separated based on a difference in structure and activity. The magnitude of pH decrease is 0.1 or more and may be 0.15 or more. The magnitude of pH decrease is less than 1, and may be 0.9 or less, 0.8 or less, 0.7 or less, 0.6 or less, 0.5 or less, 0.4 or less, 0.3 or less or 0.25 or less. The magnitude of pH decrease may be 0.1 or more and 0.6 or less, or may be 0.1 or more and 0.3 or less. When the magnitude of pH decrease is within above range, it becomes easier to separate an antibody based on a difference in structure and activity.

In an embodiment of the present invention, the pH when the step gradient starts may be a value obtained by adding a safety factor to the pH at which an antibody adsorbed onto the antibody adsorption material begin to elute. The safety factor may be appropriately set as long as it prevents a risk of elution immediately after the solution starts to flow, and for example, a constant may be used, and a value obtained by adding one to three times the standard deviation to the average value of the result measured in advance may be used.

The buffer solution used for the step gradient whose pH is adjusted by the following method can be applied to the column. First, two types of solutions with different pHs may be prepared, and an approximate expression of pH with respect to the mixing ratio of these solutions may be calculated. Next, based on the approximate expression, the two types of solutions may be mixed to obtain a desired pH and applied to the column. The buffer substance contained in the buffer solution may be a substance that has a buffering capacity in a pH range (for example, pH 3.0 or more and 6.0 or less) in which an antibody is eluted and has a plurality of pKas within this range. Examples of the buffer solution include a citrate buffer solution, an acetate buffer solution, a phosphate buffer solution, a succinate buffer solution, a phthalate buffer solution, and a glycine-hydrochloride buffer solution. Among these, when a solution containing a buffer substance having a plurality of pKas within a pH range in which antibodies elute such as a citrate ($pKa_1$=3.09, $pKa_2$=4.75) buffer solution or a succinate ($pKa_1$=4.2, $pKa_2$=5.6) buffer solution is used as a buffer solution to be applied to the column during antibody elution, the pH change due to a difference in mixing ratio becomes gentle, and handling is easier.

The flow rate of the buffer solution during the step gradient or the time to maintain the pH may be appropriately set in consideration of the size of the column filled with the antibody adsorption material and the like.

Here, conditions for changing the pH before the step gradient starts (that is, before an antibody is eluted) may be appropriately set. For example, the pH may be lowered in one step, lowered continuously (linear gradient), or lowered in steps to a pH when the step gradient starts. When the pH is lowered in steps before the antibody is eluted, the magnitude of decrease is not particularly limited.

An antibody having a desired structure and activity can be obtained by separating fractions (peaks) containing an antibody eluted in the buffer solution at each pH using the above method. Separation may be performed by a general method. Specifically, for example, a collection container may be replaced at a timing at which the pH of the buffer solution changes.

The amount of the antibody adsorption material filled into the column may be 0.1 mL or more, 0.3 mL or more, 0.5 mL or more, or 0.7 mL or more, or 0.9 mL or more. The amount of the antibody adsorption material filled into the column may be 1.4 mL or less, 1.2 mL or less, or 1.0 mL or less.

According to the method of an embodiment of the present invention, it is possible to separate a large amount of antibodies. For example, in the method of an embodiment, it is possible to separate 1 mg or more and 50 mg or less of antibody. That is, the solution containing an antibody in an embodiment may contain 1 mg or more and 50 mg or less of antibody. The solution containing an antibody in an embodiment may contain 5 mg or more and 40 mg or less of antibody.

The ratio of the mass of the antibody in the solution containing an antibody to the volume of the antibody adsorption material filled into the column may be 1 mg/1.0 mL to 50 mg/0.1 mL or 5 mg/1.0 mL to 40 mg/0.9 mL. For example, a solution containing 1 mg or more and 50 mg or less of antibody may be applied to a column filled with 0.1 mL or more and 1.0 mL or less of an antibody adsorption material, or a solution containing 5 mg or more and 40 mg or less of antibody may be applied to a column filled with 0.9 mL or more and 1.0 mL or less of an antibody adsorption material.

An antibody composition according to an aspect of the present invention contains 15 mass% or more of the antibody which is detected with a retention time of 750 minutes or longer when a monoclonal antibody solution is fractionated under the following measurement conditions, based on the total mass of the antibody composition. In other words, this antibody composition contains 15 mass% or more of the antibody which is eluted in the following citrate buffer solution with a pH of 4.0 or less when a monoclonal antibody solution is fractionated under the following measurement conditions, based on the total mass of the antibody composition. This antibody composition exhibits strong activity (for example, ADCC activity or CDC activity), and may be effective in treating cancer and the like.

(Measurement conditions)

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (filler for liquid chromatography, commercially available from Tosoh Corporation)
Flow rate: 0.07 mL/min
Solvent: phosphate buffered saline (pH 7.4)
Step gradient: add 3.73 mL each of a 50 mM citrate buffer solution (pH 6.0), a 50 mM citrate buffer solution (pH 5.5), a 50 mM citrate buffer solution (pH 5.0), a 50 mM citrate buffer solution (pH 4.8), a 50 mM citrate buffer solution (pH 4.6), a 50 mM citrate buffer solution (pH 4.4), a 50 mM citrate buffer solution (pH 4.2), a 50 mM citrate buffer solution (pH 4.0), a 50 mM citrate buffer solution (pH 3.8), a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)
Injection amount: 2,000 μL (antibody amount: 5 mg)

In the antibody composition according to one aspect of the present invention, the retention time may be 900 minutes or shorter, or 850 minutes or shorter or may be 800 minutes or shorter. The retention time may be 750 minutes or longer and 900 minutes or shorter, or 750 minutes or longer and 850 minutes or shorter or may be 750 minutes or longer and 800 minutes or shorter. The pH of the citrate buffer solution may be 3.6 to 4.0, or 3.8 to 4.0, or may be 3.4, 3.6, 3.8 or 4.0.

In the antibody composition according to one aspect of the present invention, the content of the antibody which is detected with a retention time of 750 minutes or longer when a monoclonal antibody solution is fractionated under the above measurement conditions, based on the total mass of the antibody composition, may be 20 mass% or more, 30 mass% or more, 40 mass% or more, 50 mass%, 60 mass% or more, 70 mass% or more, 80 mass% or more or 90 mass% or more. When the content is within this range, the antibody composition exhibits stronger activity (for example, ADCC activity or CDC activity), and may be more effective in treating cancer and the like.

An antibody composition according to another aspect of the present invention contains 15 mass% or more of the antibody which is detected with a retention time of shorter than 750 minutes when a monoclonal antibody solution is fractionatedunder the above measurement conditions, based on the total mass of the antibody composition. In other words, this antibody composition contains 15 mass% or more of the antibody which is eluted in the citrate buffer solution with a pH of 4.2 or more when a monoclonal antibody solution is fractionated under the above measurement conditions, based on the total mass of the antibody composition. This antibody composition exhibits weak activity (for example, ADCC activity or CDC activity), and may be effective in treating autoimmune diseases, for example, rheumatism.

In an antibody composition according to another aspect of the present invention, the retention time may be 450 minutes or longer and 550 minutes or shorter, 550 minutes or longer and 650 minutes or shorter, 650 minutes or longer and shorter than 750 minutes, 300 minutes or longer and 400 minutes or shorter, 400 minutes or longer and 480 minutes or shorter, 480 minutes or longer and 580 minutes or shorter, 580 minutes or longer and 650 minutes or shorter, 650 minutes or longer and shorter than 750 minutes, 500 minutes or longer and 600 minutes or shorter, 600 minutes or longer and 650 minutes or shorter, or 650 minutes or longer and shorter than 750 minutes. The pH of the citrate buffer solution may be 4.2 to 5.0 or 4.4 to 4.8, and may be 4.2, 4.4, 4.6, 4.8 or 5.0. As the retention time is shorter and the pH of the citrate buffer solution is higher, the antibody composition exhibits weaker activity (for example, ADCC activity or CDC activity), and may be more effective in treating autoimmune diseases, for example, rheumatism.

In an antibody composition according to another aspect of the present invention, the content of the antibody which is detected with a retention time of shorter than 750 minutes when a monoclonal antibody solution is fractionated under the above measurement conditions, based on the total mass of the antibody composition, may be 20 mass% or more, 30 mass% or more, 40 mass% or more, 50 mass%, 60 mass% or more, 70 mass% or more, 80 mass% or more, or 90 mass% or more. When the content is within this range, the antibody composition exhibits weaker activity (for example, ADCC activity or CDC activity), and may be more effective in treating autoimmune diseases, for example, rheumatism.

In addition, the antibody composition of an embodiment of the present invention may be an antibody composition containing 80 mass% or more of the antibody having sugar chains containing galactose based on the total amount of the antibody composition. This antibody composition exhibits strong activity (for example, ADCC activity or CDC activity),

and may be effective in treating cancer and the like. The content of the antibody having sugar chains containing galactose based on the total amount of the antibody composition may be 85 mass% or more, 90 mass% or more, or 95 mass% or more. When the content is within this range, the antibody composition exhibits stronger activity (for example, ADCC activity or CDC activity) and may be more effective in treating cancer and the like.

Another antibody composition of an embodiment of the present invention may be an antibody composition containing 60 mass% or more of the antibody having sugar chains containing no galactose based on the total amount of the antibody composition. It is thought that this antibody composition exhibits weak activity (for example, ADCC activity or CDC activity) and may be effective in treating autoimmune diseases, for example, rheumatism. The content of the antibody having sugar chains containing no galactose based on the total amount of the antibody composition may be 70 mass% or more, 80 mass% or more, or 90 mass% or more. When the content is within this range, the antibody composition exhibits weaker activity (for example, ADCC activity or CDC activity), and may be more effective in treating autoimmune diseases, for example, rheumatism.

A concentrate according to one aspect of the present invention is a concentrate of an antibody solution, and the antibody solution and the concentrate contain the antibody which is detected with a retention time of 750 minutes or longer when a monoclonal antibody solution is fractionated under the above measurement conditions, and the content of the antibody based on the total amount of the concentrate is at least twice the content of the antibody based on the total amount of the antibody solution. In other words, the concentrate contains the antibody which is eluted in the citrate buffer solution with a pH of 4.0 or less when a monoclonal antibody solution is fractionated under the above measurement conditions, and the content of the antibody based on the total amount of the concentrate is at least twice the content of the antibody based on the total amount of the antibody solution. The concentrate exhibits strong activity (for example, ADCC activity or CDC activity) and may be effective in treating cancer and the like.

In a concentrate according to one aspect of the present invention, the retention time may be 900 minutes or shorter, 850 minutes or shorter, or 800 minutes or shorter. The retention time may be 750 minutes or longer and 900 minutes or shorter, 750 minutes or longer and 850 minutes or shorter, or 750 minutes or longer and 800 minutes or shorter. The pH of the citrate buffer solution may be 3.6 to 4.0, or 3.8 to 4.0, or may be 3.4, 3.6, 3.8 or 4.0.

In a concentrate according to one aspect of the present invention, the content of the antibody based on the total amount of the concentrate may be at least 2.5 times, 3 times, or 3.5 times the content of the antibody based on the total amount of the antibody solution. In this case, the concentrate exhibits stronger activity (for example, ADCC activity or CDC activity) and may be more effective in treating cancer and the like.

A concentrate according to another aspect of the present invention is a concentrate of an antibody solution, the antibody solution and the concentrate contains the antibody which is detected with a retention time of shorter than 750 minutes when a monoclonal antibody solution is fractionated under the above measurement conditions, and the content of the antibody based on the total amount of the concentrate is at least twice the content of the antibody based on the total amount of the antibody solution. In other words, the concentrate contains the antibody which is eluted in the citrate buffer solution with a pH of 4.2 or more when a monoclonal antibody solution is fractionated under the above measurement conditions, and the content of the antibody based on the total amount of the concentrate is at least twice the content of the antibody based on the total amount of the antibody solution. The concentrate exhibits weak activity (for example, ADCC activity or CDC activity) and may be effective in treating autoimmune diseases, for example, rheumatism.

In a concentrate according to another aspect of the present invention, the retention time may be 450 minutes or longer and 550 minutes or shorter, 550 minutes or longer and 650 minutes or shorter, 650 minutes or longer and shorter than 750 minutes, 300 minutes or longer and 400 minutes or shorter, 400 minutes or longer and 480 minutes or shorter, 480 minutes or longer and 580 minutes or shorter, 580 minutes or longer and 650 minutes or shorter, 650 minutes or longer and shorter than 750 minutes, 500 minutes or longer and 600 minutes or shorter, 600 minutes or longer and 650 minutes or shorter, or 650 minutes or longer and shorter than 750 minutes. The pH of the citrate buffer solution may be 4.2 to 5.0 or 4.4 to 4.8, and may be 4.2, 4.4, 4.6, 4.8 or 5.0. As the retention time is shorter and the pH of the citrate buffer solution is higher, the concentrate exhibits weaker activity (for example, ADCC activity or CDC activity) and may be more effective in treating autoimmune diseases, for example, rheumatism.

In a concentrate according to another aspect of the present invention, the content of the antibody based on the total amount of the concentrate may be at least 2.5 times, 3 times, or 3.5 times the content of the antibody based on the total amount of the antibody solution. In this case, the concentrate exhibits weaker activity (for example, ADCC activity or CDC activity) and may be more effective in treating autoimmune diseases, for example, rheumatism.

All of these antibody compositions and concentrates can be obtained by the separation method of an embodiment of the present invention. The structure of sugar chains of an antibody can be analyzed by known methods such as liquid chromatography mass spectrometry (LC/MS).

[Examples]

Embodiments of the present invention will be described in more detail with reference to examples and comparative examples, but the present invention is not limited to these examples.

(Reference Example 1) Preparation of antibody adsorption material

1 g of a porous hydrophilic vinyl polymer for an adsorption material (TOYOPEARL (filler for liquid chromatography, commercially available from Tosoh Corporation), volume-based Mean particle Diameter: 45 μm) was collected as a suction dry gel, the hydroxy group on the gel surface was activated with an iodoacetyl group, and 30 mg of Fc-binding protein FcR36i_Cys (SEQ ID NO: 2) disclosed in PCT International Publication No. WO2019/083048 was then reacted to obtain an antibody adsorption material FcR36i_Cys immobilized gel.

In FcR36i_Cys (SEQ ID NO: 2), a sequence from the 1st methionine (Met) to the 22nd alanine (Ala) is a PelB signal peptide, a sequence from the 24th glycine (Gly) to the 199th glutamine (Gln) is an amino acid sequence of Fc-binding protein FcR36i, and a sequence from the 200th glycine (Gly) to the 207th glycine (Gly) is a cysteine tag sequence. FcR36i is a polypeptide including an extracellular region of natural human FcγRIIIa (the amino acid sequence from the 17th glycine (Gly) to the 192nd glutamine (Gln) in the amino acid sequence set forth as SEQ ID NO: 1), and having amino acid substitutions of Glu21Gly (this notation indicates that the 21st glutamic acid in SEQ ID NO: 1 (the 28th glutamic acid in SEQ ID NO: 2) is replaced with glycine, the same applies hereinafter), Leu23Met, Val27Glu, Phe29Ile, Gln33Pro, Tyr35Asn, Lys40Gln, Gln48Arg, Tyr51His, Glu54Asp, Asn56Asp, Ser65Arg, Ser68Pro, Tyr74Phe, Phe75Ile, Ala78Ser, Thr80Ser, Asn92Ser, Val117Glu, Lys119Val, Glu121Gly,Asp122Glu, Ly s 13 2Arg, Thr140Met, Tyr141Phe, Gly147Val, Tyr158Val, Lys165Glu, Phe171Ser, Val176Ile, Ser178Arg, Asn180Lys, Glu184Gly, Thr185Ala, Asn187Asp, and Ile 1 90Val in the amino acid residues in the region.

The antibody adsorption material obtained in Reference Example 1 had good alkali resistance. More specifically, even after cleaning in place (CIP) of the antibody adsorption material was performed 200 times with a 0.1 M NaOH solution, 90% of the amount of antibody adsorbed was maintained compared to before washing. In addition, the antibody adsorption material obtained in Reference Example 1 had a dynamic binding capacity (DBC) of 40 g/L or more.

(Reference Example 2) Preparation of buffer solution for antibody separation

A 50 mM citrate aqueous solution and a 50 mM trisodium citrate aqueous solution were produced as stock solutions for a citrate buffer solution used for antibody separation. The two types of aqueous solutions produced were mixed at an arbitrary ratio, and the pH of the obtained mixed solution (citrate buffer solution) was measured. An approximate curve was created from the measured results (FIG. 1), and the mixing ratio of two types of aqueous solutions at a specific pH was calculated. In the graph shown in FIG. 1, the horizontal axis represents the proportion of the 50 mM trisodium citrate (Citrate-3Na) aqueous solution based on the total mass of the 50 mM citrate aqueous solution and the 50 mM trisodium citrate aqueous solution, and the vertical axis represents pH.

Table 1 shows the relationship between the mixing ratio of the 50 mM citrate aqueous solution (50 mM Citric acid) and the 50 mM trisodium citrate aqueous solution (50 mM Citrate-3Na) obtained from the approximate curve (FIG. 1) and the pH.

[Table 1]

| pH | Mixing ratio [mass%] | |
|---|---|---|
| | 50 mM Citric acid | 50 mM Citrate-3Na |
| 6.0 | 10.8 | 89.2 |
| 5.5 | 22.9 | 77.1 |
| 5.0 | 35.0 | 65.0 |
| 4.8 | 39.8 | 60.2 |
| 4.6 | 44.7 | 55.3 |
| 4.5 | 47.1 | 52.9 |
| 4.4 | 49.5 | 50.5 |
| 4.2 | 54.3 | 45.7 |
| 4.0 | 59.1 | 40.9 |

(continued)

| pH | Mixing ratio [mass%] | |
|---|---|---|
| | 50 mM Citric acid | 50 mM Citrate-3Na |
| 3.8 | 64.0 | 36.0 |
| 3.6 | 68.8 | 31.2 |
| 3.5 | 71.2 | 28.8 |
| 3.4 | 73.6 | 26.4 |
| 3.0 | 83.3 | 16.7 |

(Example 1) Separation of an antibody using antibody adsorption material-filled column

(1) The antibody adsorption material (FcR36i_Cys immobilized gel) produced in Reference Example 1 was filled into a φ5.0 mm×50 mm Tricorn column (Tricorn 5/50, commercially available from Cytiva) to produce an FcR36i_Cys column with a gel volume of 0.373 mL.
(2) The FcR36i_Cys column produced in (1) was connected to AKTA avant 150 (commercially available from Cytiva), and 5 column volume (hereinafter referred to as "CV"; 1 CV=0.373 mL) of a phosphate buffered saline (pH 7.4) (hereinafter referred to as PBS) was then applied to the column at a flow rate of 0.07 mL/min, and thus the column was equilibrated.
(3) 5 mg of monoclonal antibodies (Rituxan, human-mouse chimeric monoclonal antibody, commercially available from Zenyaku Holdings Co., Ltd.) diluted with PBS to be 2.5 mg/mL was applied as the antibody to the FcR36i_Cys column at a flow rate of 0.07 mL/min.
(4) The column was washed by applying 20 CV of PBS to the FcR36i_Cys column while maintaining the flow rate at 0.07 mL/min.
(5) To the FcR36i_Cys column, 10 CV each of a 50 mM citrate buffer solution (pH 6.0), a 50 mM citrate buffer solution (pH 5.5), and a 50 mM citrate buffer solution (pH 5.0) was applied in that order, and the pH was lowered in steps (step gradient). Citrate buffer solutions with each pH were prepared by mixing a 50 mM citrate aqueous solution and a 50 mM trisodium citrate aqueous solution based on the mixing ratio determined in Reference Example 2 (the same applies to citrate buffer solutions with other pHs used in (6) and later). Here, the step gradient from pH 6.0 to pH 5.0 is indicated by a solid line in FIG. 2.
(6) To the FcR36i_Cys column, 10 CV each of a 50 mM citrate buffer solution (pH 4.5), a 50 mM citrate buffer solution (pH 4.0), a 50 mM citrate buffer solution (pH 3.5) and a 50 mM citrate buffer solution (pH 3.0) was applied in that order. In this manner, a step gradient in which the pH was lowered in steps decrease of 0.5 was implemented and an separation pattern of the FcR36i_Cys column was obtained. In addition, in (5) and (6), fractions containing a monoclonal antibody eluted from the antibody adsorption material (FcR36i_Cys immobilized gel) were separated for each applied buffer solution at each pH. Here, a step gradient in which the pH was lowered in steps by 0.5 from 5.0 is indicated by a dashed line in FIG. 2.
(7) The fractions separated in (5) and (6) were dialyzed against PBS for 16 hours, and the solvent was replaced.
(8) A TSKgel FcR-IIIA-NPR column (commercially available from Tosoh Corporation; hereinafter simply referred to as FcRIIIA column) was connected to a high performance liquid chromatography device (commercially available from Shimadzu Corporation). Then, 30 CV of a 20 mM acetic acid-sodium acetate buffer solution containing 50 mM sodium chloride (pH 5.0) (hereinafter simply referred to as acetate buffer solution) was applied to the FcRIIIA column at a flow rate of 0.6 mL/min, and thus the FcRIIIA column was equilibrated.
(9) 5 μg of the monoclonal antibody solution (fractions separated in (5) and (6)) for which the solvent was replaced in (7) was applied to the FcRIIIA column at a flow rate of 0.6 mL/min.
(10) After washing with an acetate buffer solution for 2 minutes while maintaining the flow rate at 0.6 mL/min, the adsorbed monoclonal antibodies were eluted with a pH gradient (a linear gradient in which a 10 mM glycine-hydrochloric acid buffer solution (pH 3.0) reaches 100% in 28 minutes) of a 10 mM glycine-hydrochloric acid buffer solution (pH 3.0), and an separation pattern of the FcRIIIA column was obtained.

FIG. 3 shows the separation pattern of the FcR36i_Cys column and FIG. 4 shows the separation pattern of the FcRIIIA column. In FIG. 3, the vertical axis (left) represents absorbance at 280 nm, the vertical axis (right) represents set pH (the pH of the citrate buffer solution), and the horizontal axis represents time (minutes). In FIG. 4, the vertical

axis represents absorbance at 280 nm and the horizontal axis represents time (minutes). When the antibodies adsorbed onto the antibody adsorption material were eluted with a step gradient in which the pH was lowered in steps decrease of 0.5, the pattern was divided into two peaks (F1 and F2) (FIG. 3). When the F1 fraction and the F2 fraction in FIG. 3 were separated and analyzed using the FcRIIIA column, it was found that, in the F1 fraction, antibodies were mainly eluted at the first peak (1st), and the fraction contained many antibodies having structures of sugar chain with weak antibody-dependent cellular cytotoxicity (ADCC) activity (FIG. 4). Thus, according to the method of an embodiment of the present invention, it was found that 5 mg of antibody could be fractionated based on a difference in ADCC activity. Here, in FIG. 4 (and FIGS. 6, 8, 10, 12, 14, and 16 to be described below), an antibody having structures of sugar chain with weak antibody-dependent cellular cytotoxicity (ADCC) activity was separated at the first peak (1st), an antibody having structures of sugar chain with strong ADCC activity was separated at the third peak (3rd), and an antibody having structures of sugar chain exhibiting intermediate ADCC activity was separated at the second peak (2nd).

(Example 2) Separation of an antibody using antibody adsorption material-filled column

Separation patterns of the FcR36i_Cys column and the FcRIIIA column were obtained in the same method as in Example 1 except that, in (6) of Example 1, to the FcR36i_Cys column, 10 CV each of a 50 mM citrate buffer solution (pH 4.8), a 50 mM citrate buffer solution (pH 4.6), a 50 mM citrate buffer solution (pH 4.4), a 50 mM citrate buffer solution (pH 4.2), a 50 mM citrate buffer solution (pH 4.0), a 50 mM citrate buffer solution (pH 3.8), a 50 mM citrate buffer solution (pH 3.6) and a 50 mM citrate buffer solution (pH 3.4) was applied in that order, and thus a step gradient in which the pH was lowered in steps decrease of 0.2 was implemented. The step gradient implemented in this example is indicated by a dotted line in FIG. 2.

FIG. 5 shows the separation pattern of the FcR36i_Cys column, and FIG. 6 shows the separation pattern of the FcRIIIA column. In FIG. 5, the vertical axis (left) represents absorbance at 280 nm, the vertical axis (right) represents set pH (the pH of the citrate buffer solution), and the horizontal axis represents time (minutes). In FIG. 6, the vertical axis represents absorbance at 280 nm, and the horizontal axis represents time (minutes). When the antibodies adsorbed onto the antibody adsorption material were eluted with a step gradient in which the pH was lowered in steps decrease of 0.2, the pattern was divided into a larger number of peaks than in Example 1 (a magnitude of decrease of 0.5) (FIG. 5). In addition, when the F 1 fraction to the F4 fraction in FIG. 5 were separated and analyzed using the FcRIIIA column, it was found that the F1 fraction mainly contained the first peak (1st), that is, an antibody having structures of sugar chain with weak ADCC activity, the F3 fraction mainly contained the second peak (2nd), that is, an antibody having structures of sugar chain exhibiting intermediate ADCC activity, and the F4 fraction mainly contained the third peak (3rd), that is, an antibody having structures of sugar chain with strong ADCC activity (FIG. 6). Based on the above results, it was found that, when the magnitude of pH decrease was changed from 0.5 to 0.2, 5 mg of antibody could be more easily fractionated based on a difference in structure of sugar chain (here, a difference in ADCC activity).

(Comparative Example 1)

Separation patterns of the FcR36i_Cys column and the FcRIIIA column were obtained in the same method as in Example 1 except that, in (6) of Example 1, to the FcR36i_Cys column, 10 CV each of a 50 mM citrate buffer solution (pH 4.0) and a 50 mM citrate buffer solution (pH 3.0) was applied in that order, and thus a step gradient in which the pH was lowered in steps decrease of 1.0 was implemented. The step gradient implemented in this comparative example is indicated by a solid line in FIG. 2.

FIG. 7 shows the separation pattern of the FcR36i_Cys column and FIG. 8 shows the separation pattern of the FcRIIIA column. In FIG. 7, the vertical axis (left) represents absorbance at 280 nm, the vertical axis (right) represents set pH (the pH of the citrate buffer solution), and the horizontal axis represents time (minutes). In FIG. 8, the vertical axis represents absorbance at 280 nm, and the horizontal axis represents time (minutes). When the antibodies adsorbed onto the antibody adsorption material were eluted with a step gradient in which the pH was lowered in steps decrease of 1.0, unlike Example 1 (a magnitude of decrease 0.5) and Example 2 (a magnitude of decrease 0.2), there was one peak (FIG. 7). Therefore, it was found that, with a magnitude of decrease 1.0, antibodies could not be separated based on a difference in structure of sugar chain (here, a difference in ADCC activity) using the FcR36i_Cys column.

To summarize the results of Example 1, Example 2 and Comparative Example 1, it was found that, when an antibody having desired structures of sugar chain (here, desired ADCC activity) was separated using an antibody adsorption material containing a porous carrier and an Fc-binding protein immobilized on the carrier (FcR36i_Cys column), it was necessary to elute the antibody adsorbed onto the antibody adsorption material with a step gradient in which the pH was lowered in steps decrease of 0.1 or more and less than 1. In addition, comparing Example 1 and Example 2, it was found that, when the magnitude of decrease was set to 0.1 or more and 0.3 or less, an antibody having desired structures of sugar chain (here, desired ADCC activity) could be more easily separated.

(Example 3) Separation of antibody using antibody adsorption material-filled column

Separation patterns of the FcR36i_Cys column and the FcRIIIA column were obtained in the same method as in Example 1 except that, in (3) of Example 1, an Actemra (commercially available from Roche; anti-interleukin 6 monoclonal antibody; addition amount 5 mg) solution was used in place of the monoclonal antibody solution applied to the FcR36i_Cys column. The step gradient implemented in this example is indicated by a dashed line in FIG. 2.

(Example 4) Separation of antibody using antibody adsorption material-filled column

Separation patterns of the FcR36i_Cys column and the FcRIIIA column were obtained in the same method as in Example 1 except that, in (3) of Example 1, an Actemra (commercially available from Roche; anti-interleukin 6 monoclonal antibody; addition amount 5 mg) solution was used in place of the monoclonal antibody solution applied to the FcR36i_Cys column, and the step gradient implemented in (6) of Example 1 was performed by the method described in Example 2. The step gradient implemented in this example is indicated by a dotted line in FIG. 2.

FIG. 9 and FIG. 10 show separation patterns of the FcR36i_Cys column and the FcRIIIA column in Example 3 (magnitude of pH decrease: 0.5). In FIG. 9, the vertical axis (left) represents absorbance at 280 nm, the vertical axis (right) represents set pH (the pH of the citrate buffer solution), and the horizontal axis represents time (minutes). In FIG. 10, the vertical axis represents absorbance at 280 nm, and the horizontal axis represents time (minutes). FIG. 11 and FIG. 12 show separation patterns of the FcR36i_Cys column and the FcRIIIA column in Example 4 (magnitude of pH decrease: 0.2). In FIG. 11, the vertical axis (left) represents absorbance at 280 nm, the vertical axis (right) represents set pH (the pH of the citrate buffer solution), and the horizontal axis represents time (minutes). In FIG. 12, the vertical axis represents absorbance at 280 nm, and the horizontal axis represents time (minutes). When the magnitude of pH decrease was 0.5 (Example 3), the pattern was divided into three peaks (F1 to F3) (FIG. 9). In addition, when the three peak fractions were separated and analyzed using the FcRIIIA column, it was found that the F1 fraction and the F2 fraction mainly contained the first peak (1st), that is, an antibody having structures of sugar chain with weak ADCC activity (FIG. 10). When the magnitude of pH decrease was 0.2 (Example 4), the pattern was divided into a plurality of peaks (FIG. 11). When the F1 fraction to the F6 fraction in FIG. 11 were separated and analyzed using the FcRIIIA column, it was found that the F1 to F4 fractions mainly contained the first peak (1st), that is, an antibody having structures of sugar chain with weak ADCC activity, the F5 fraction mainly contained the second peak (2nd), that is, an antibody having structures of sugar chain exhibiting intermediate ADCC activity, and the F6 fraction mainly contained the third peak (3rd), that is, an antibody having structures of sugar chain with strong ADCC activity (FIG. 12).

According to an embodiment, it was found that, even if the monoclonal antibody was changed from Rituxan to Actemra, when the antibody adsorbed onto the antibody adsorption material were eluted with a step gradient in which the pH was lowered in steps decrease of 0.1 or more and less than 1, a large amount of antibodies could be fractionated based on a difference in structure of sugar chain (here, a difference in ADCC activity), and could be more easily fractionated when the magnitude of decrease was 0.1 or more and 0.3 or less.

(Example 5) Separation of an antibody using antibody adsorption material-filled column

Separation patterns of the FcR36i_Cys column and the FcRIIIA column were obtained in the same method as in Example 1 except that, in (3) of Example 1, an Erbitux (commercially available from Merck Serono; human/mouse chimerized monoclonal antibody; addition amount 5 mg) solution was used in place of the monoclonal antibody solution applied to the FcR36i_Cys column. The step gradient implemented in this example is indicated by a dashed line in FIG. 2.

(Example 6) Separation of an antibody using antibody adsorption material-filled column

Separation patterns of the FcR36i_Cys column and the FcRIIIA column were obtained in the same method as in Example 1 except that, in (3) of Example 1, an Erbitux (commercially available from Merck Serono; human/mouse chimerized monoclonal antibody; addition amount 5 mg) solution was used in place of the monoclonal antibody solution applied to the FcR36i_Cys column, and the step gradient implemented in Example 1 (6) was performed by the method described in Example 2. The step gradient implemented in this example is indicated by a dotted line in FIG. 2.

FIG. 13 and FIG. 14 show separation patterns of the FcR36i_Cys column and the FcRIIIA column in Example 5 (a pH decrease by 0.5). In FIG. 13, the vertical axis (left) represents absorbance at 280 nm, the vertical axis (right) represents set pH (the pH of the citrate buffer solution), and the horizontal axis represents time (minutes). In FIG. 14, the vertical axis represents absorbance at 280 nm and the horizontal axis represents time (minutes). FIG. 15 and FIG. 16 show separation patterns of the FcR36i_Cys column and the FcRIIIA column in Example 6 (a pH decrease by 0.2). In FIG. 15, the vertical axis (left) represents absorbance at 280 nm, the vertical axis (right) represents set pH (the pH of the citrate buffer solution), and the horizontal axis represents time (minutes). In FIG. 16, the vertical axis represents

absorbance at 280 nm, and the horizontal axis represents time (minutes). When the magnitude of pH decrease was 0.5 (Example 5), the pattern was divided into two peaks (F1 and F2) (FIG. 13). When the F1 fraction and the F2 fraction in FIG. 13 were separated and analyzed using the FcRIIIA column, it was found that the F1 fraction contained the first peak (1st), that is, many antibodies having structures of sugar chain with weak ADCC activity (FIG. 14). When the magnitude of pH decrease was 0.2 (Example 6), the pattern was divided into four peaks (F1 to F4) (FIG. 15). When these four peak fractions were separated and analyzed using the FcRIIIA column, it was found that the F2 fraction mainly contained the first peak (1st), that is, an antibody having structures of sugar chain with weak ADCC activity, the F3 fraction mainly contained the first peak and the second peak (2nd), that is, an antibody having structures of sugar chain with intermediate or lower ADCC activity, and the F4 fraction mainly contained the second peak and the third peak (3rd), that is, an antibody having structures of sugar chain with intermediate or higher ADCC activity (FIG. 16).

According to an embodiment, it was found that, even if the monoclonal antibody was changed from Rituxan to Erbitux, when the antibody adsorbed onto the antibody adsorption material were eluted with a step gradient in which the pH was lowered in steps decrease of 0.1 or more and less than 1, a large amount of antibodies could be fractionated based on a difference in structure of sugar chain (here, a difference in ADCC activity), and could be more easily fractionated when the magnitude of decrease was 0.1 or more and 0.3 or less.

(Example 7) Separation of an antibody using antibody adsorption material-filled column

<1> The antibody adsorption material (FcR36i_Cys immobilized gel) produced in Reference Example 1 was filled into a φ5.0 mm×50 mm Tricorn column (Tricorn 5/50, commercially available from Cytiva) to produce an FcR36i_Cys column with a gel volume of 0.982 mL.

<2> The FcR36i_Cys column produced in <1> was connected to AKTA avant 150 (commercially available from Cytiva) and 5 CV of PBS (pH 7.4) was then applied to the column at a flow rate of 0.20 mL/min, and thus the column was equilibrated.

<3> 5 mg of monoclonal antibody (Rituxan, human-mouse chimeric monoclonal antibody, commercially available from Zenyaku Holdings Co., Ltd.) diluted with PBS to be 5.0 mg/mL was applied to the FcR36i_Cys column at a flow rate of 0.20 mL/min.

<4> The column was washed by applying 20 CV of PBS to the FcR36i_Cys column while maintaining the flow rate at 0.20 mL/min.

<5> To the FcR36i_Cys column, 10 CV each of a 50 mM citrate buffer solution (pH 6.0), a 50 mM citrate buffer solution (pH 5.5), a 50 mM citrate buffer solution (pH 5.0) and a 50 mM citrate buffer solution (pH 4.8) was applied in that order, and the pH was lowered in steps (step gradient).

<6> To the FcR36i_Cys column, 30 CV each of a 50 mM citrate buffer solution (pH 4.6), a 50 mM citrate buffer solution (pH 4.4), a 50 mM citrate buffer solution (pH 4.2), a 50 mM citrate buffer solution (pH 4.0) and a 50 mM citrate buffer solution (pH 3.8) was applied in that order, and 10 CV each of a 50 mM citrate buffer solution (pH 3.6) and a 50 mM citrate buffer solution (pH 3.4) was then applied in that order. In this manner, a step gradient in which the pH was lowered in steps decrease of 0.2 was implemented, and an separation pattern of the FcR36i_Cys column was obtained. In addition, in <5> and <6>, fractions containing monoclonal antibodies eluted from the antibody adsorption material (FcR36i_Cys immobilized gel) were separated for each applied buffer solution at each pH. The step gradient implemented in this example is indicated by a solid line in FIG. 17.

<7> The fractions separated in <5> and <6> were dialyzed against PBS for 16 hours, and the solvent was replaced.

<8> The FcRIIIA column was connected to a high performance liquid chromatography device (commercially available from Shimadzu Corporation). Then, 30 CV of a 20 mM acetic acid-sodium acetate buffer solution containing 50 mM sodium chloride (pH 5.0) was applied to the FcRIIIA column at a flow rate of 0.6 mL/min, and thus the FcRIIIA column was equilibrated.

<9> 5 μg of the monoclonal antibody solution (fractions separated in <5> and <6>) for which the solvent was replaced in <7> was applied to the FcRIIIA column at a flow rate of 0.6 mL/min.

<10> After washing with an acetate buffer solution for 2 minutes while maintaining the flow rate at 0.6 mL/min, the adsorbed monoclonal antibodies were eluted with a pH gradient (a linear gradient in which a 10 mM glycine-hydrochloric acid buffer solution (pH 3.0) reaches 100% in 28 minutes) of a 10 mM glycine-hydrochloric acid buffer solution (pH 3.0), and an separation pattern of the FcRIIIA column was obtained.

FIG. 18 shows the separation pattern of the FcR36i_Cys column, and FIG. 19 shows the separation pattern of the FcRIIIA column. In FIG. 18, the vertical axis (left) represents absorbance at 280 nm, the vertical axis (right) represents set pH (the pH of the citrate buffer solution), and the horizontal axis represents time (minutes). When the an antibody adsorbed onto the antibody adsorption material was eluted with a step gradient in which the pH was lowered in steps

decrease of 0.2, the pattern was divided into three peaks (F1, F2 and F3) (FIG. 18). When the F1 fraction, the F2 fraction and the F3 fraction in FIG. 18 were separated and analyzed using the FcRIIIA column, it was found that the F1 fraction mainly contained the first peak (1st), that is, an antibody having structures of sugar chain with weak ADCC activity, the F2 fraction mainly contained the second peak (2nd), that is, an antibody having structures of sugar chain exhibiting intermediate ADCC activity, and the F3 fraction mainly contained the third peak (3rd), that is, an antibody having structures of sugar chain with strong ADCC activity (FIG. 19).

(Example 8) Separation of an antibody using antibody adsorption material-filled column

Separation patterns of the FcR36i_Cys column and the FcRIIIA column were obtained in the same method as in Example 7 except that the amount of monoclonal antibodies (Rituxan, human-mouse chimeric monoclonal antibody, commercially available from Zenyaku Holdings Co., Ltd.) applied to the FcR36i_Cys column in <3> in Example 7 was 10 mg. The step gradient implemented in this example is shown in FIG. 17.

FIG. 20 shows the separation pattern of the FcR36i_Cys column, and FIG. 21 shows the separation pattern of the FcRIIIA column. In FIG. 20, the vertical axis (left) represents absorbance at 280 nm, the vertical axis (right) represents set pH (the pH of the citrate buffer solution), and the horizontal axis represents time (minutes). When the an antibody adsorbed onto the antibody adsorption material was eluted with a step gradient in which the pH was lowered in steps decrease of 0.2, the pattern was divided into four peaks (F1 to F4) (FIG. 20). When the F1 fraction to the F4 fraction in FIG. 20 were separated and analyzed using the FcRIIIA column, it was found that the F1 and F2 fractions mainly contained the first peak (1st), that is, an antibody having structures of sugar chain with weak ADCC activity, the F3 fraction mainly contained the second peak (2nd) and the third peak (3rd), that is, an antibody having structures of sugar chain exhibiting intermediate or higher ADCC activity, and the F4 fraction mainly contained the third peak (3rd), that is, an antibody having structures of sugar chain with strong ADCC activity (FIG. 21).

(Example 9) Separation of an antibody using antibody adsorption material-filled column

Separation patterns of the FcR36i_Cys column and the FcRIIIA column were obtained in the same method as in Example 7 except that the amount of monoclonal antibodies (Rituxan, human-mouse chimeric monoclonal antibody, commercially available from Zenyaku Holdings Co., Ltd.) applied to the FcR36i_Cys column in <3> in Example 7 was 20 mg. The step gradient implemented in this example is shown in FIG. 17.

FIG. 22 shows the separation pattern of the FcR36i_Cys column, and FIG. 23 shows the separation pattern of the FcRIIIA column. In FIG. 22, the vertical axis (left) represents absorbance at 280 nm, the vertical axis (right) represents set pH (the pH of the citrate buffer solution), and the horizontal axis represents time (minutes). When the antibody adsorbed onto the antibody adsorption material was eluted with a step gradient in which the pH was lowered in steps decrease of 0.2, the pattern was divided into four peaks (F1 to F4) (FIG. 22). When the F1 fraction to the F4 fraction in FIG. 22 were separated and analyzed using the FcRIIIA column, it was found that the F1 and F2 fractions mainly contained the first peak (1st), that is, an antibody having structures of sugar chain with weak ADCC activity, the F3 fraction mainly contained the second peak (2nd) and the third peak (3rd), that is, an antibody having structures of sugar chain exhibiting intermediate or higher ADCC activity, and the F4 fraction mainly contained the third peak (3rd), that is, an antibody having structures of sugar chain with strong ADCC activity (FIG. 23).

(Example 10) Separation of an antibody using antibody adsorption material-filled column

Separation patterns of the FcR36i_Cys column and the FcRIIIA column were obtained in the same method as in Example 7 except that the amount of monoclonal antibodies (Rituxan, human-mouse chimeric monoclonal antibody, commercially available from Zenyaku Holdings Co., Ltd.) applied to the FcR36i_Cys column in <3> in Example 7 was 30 mg. The step gradient implemented in this example is shown in FIG. 17.

FIG. 24 shows the separation pattern of the FcR36i_Cys column, and FIG. 25 shows the separation pattern of the FcRIIIA column. In FIG. 24, the vertical axis (left) represents absorbance at 280 nm, the vertical axis (right) represents set pH (the pH of the citrate buffer solution), and the horizontal axis represents time (minutes). When the antibody adsorbed onto the antibody adsorption material was eluted with a step gradient in which the pH was lowered in steps decrease of 0.2, the pattern was divided into six peaks (F1 to F6) (FIG. 24). When the F1 fraction to the F6 fraction in FIG. 24 were separated and analyzed using the FcRIIIA column, it was found that the F1 to F3 fractions mainly contained the first peak (1st), that is, an antibody having structures of sugar chain with weak ADCC activity, the F4 fraction mainly contained the first peak (1st) and the second peak (2nd), antibodies having structures of sugar chain exhibiting intermediate or lower ADCC activity, the F5 fraction mainly contained the second peak (2nd), that is, an antibody having structures of sugar chain exhibiting intermediate ADCC activity, and the F6 fraction mainly contained the third peak (3rd), that is, an antibody having structures of sugar chain with strong ADCC activity (FIG. 25).

(Example 11) Separation of an antibody using antibody adsorption material-filled column

Separation patterns of the FcR36i_Cys column and the FcRIIIA column were obtained in the same method as in Example 7 except that the amount of monoclonal antibodies (Rituxan, human-mouse chimeric monoclonal antibody, commercially available from Zenyaku Holdings Co., Ltd.) applied to the FcR36i_Cys column in <3> in Example 7 was 40 mg. The step gradient implemented in this example is shown in FIG. 17.

FIG. 26 shows the separation pattern of the FcR36i_Cys column and FIG. 27 shows the separation pattern of the FcRIIIA column. In FIG. 26, the vertical axis (left) represents absorbance at 280 nm, the vertical axis (right) represents set pH (the pH of the citrate buffer solution), and the horizontal axis represents time (minutes). When the antibody adsorbed onto the antibody adsorption material were eluted with a step gradient in which the pH was lowered in steps decrease of 0.2, the pattern was divided into a plurality of peaks (FIG. 26). When the F1 fraction to the F6 fraction in FIG. 26 were separated and analyzed using the FcRIIIA column, it was found that the F1 and F2 fractions mainly contained the first peak (1st), that is, an antibody having structures of sugar chain with weak ADCC activity, the F3 and F4 fractions mainly contained the second peak (2nd), that is, an antibody having structures of sugar chain exhibiting intermediate ADCC activity, the F5 fraction mainly contained the second peak (2nd) and the third peak (3rd), that is, an antibody having structures of sugar chain exhibiting intermediate or higher ADCC activity, and the F6 fraction mainly contained the third peak (3rd), that is, an antibody having structures of sugar chain with strong ADCC activity (FIG. 27).

To summarize the above, it was found that, even if the amount of monoclonal antibodies applied was changed from a range of 5 mg to 40 mg, when the antibody adsorbed onto the antibody adsorbing material was eluted with a step gradient in which the pH was lowered in steps decrease of 0.2, the antibody could be separated based on a difference in structure of sugar chain (for example, ADCC activity).

(Example 12) Structure of sugar chain analysis of an antibody separated by the method of an embodiment of the present invention

<1> A RapiGest SF solution (bundled in Glyco Works RapiFluor-MS N-Glycan kit [commercially available from Waters]) was added to the F2 fraction separated in Example 7 in FIG. 18 and the F1, F2 and F4 fractions separated in Example 8 in FIG. 20, a heat treatment was then performed at 90°C for 3 minutes, and thus the antibodies contained in these fractions were modified. The antibodies after the heat treatment were treated with Rapid PNGaseF (commercially available from New England Biolabs), and sugar chains of the antibodies were cut out.

<2> The sugar chains cut out in <1> were fluorescently labeled by adding a RapiFlour-MS reagent solution (bundled in kit commercially available from Waters). The labeled sugar chains were purified using a hydrophilic interaction liquid chromatography (HILIC) column.

<3> The fluorescently labeled sugar chains obtained in <2> were separated using a Glycan BEH Amide column (commercially available from Waters), and molecular weight information of the sugar chains was then obtained using a mass spectrometer. The structure of sugar chain was analyzed by comparing the retention time in the chromatogram obtained using the Glycan BEH Amide column.

FIG. 28 and Table 2 show the obtained structure of sugar chain analysis results, and Table 3 shows schematic views of the structures of sugar chain. In the graph shown in FIG. 28, the vertical axis represents the proportions of structures of sugar chain (peak area proportion of each structure of sugar chain). The sugar names (abbreviations) listed along the horizontal axis are the same as the abbreviations used in Table 3.

According to FIG. 28 and Table 2, in the antibodies contained in the F4 fraction (eluted with a citrate buffer solution with a pH of 4.0) in FIG. 20, the content of antibodies having structures of sugar chain containing galactose at the non-reducing terminal (for example, GIFa and G2F) was high, and the content of antibodies having structures of sugar chain containing no galactose at the non-reducing terminal (for example, G0F) was low. On the other hand, in the antibodies contained in the F1 fraction (eluted with a citrate buffer solution with a pH of 4.6) in FIG. 20, the antibodies contained in the F2 fraction (eluted with a citrate buffer solution with a pH of 4.4) in FIG. 20, and the antibodies contained in the F2 fraction (eluted with a citrate buffer solution with a pH of 4.2) in FIG. 18, the content of antibodies having structures of sugar chain containing galactose at the non-reducing terminal and the content of antibodies having structures of sugar chain containing no galactose at the non-reducing terminal were not significantly changed. This suggests that, when an antibody eluted with a citrate buffer solution with a pH of 4.0 or less was collected, it was possible to obtain antibodies including a large amount of antibodies having structures of sugar chain containing galactose at the non-reducing terminal. That is, it was found that the antibody contained in the solution could be separated by the separation method of an embodiment of the present invention based on a difference in structure of sugar chain, specifically, based on the presence or absence of galactose residues at the non-reducing terminal.

In addition, it was shown that, in the antibodies contained in the F2 fraction in FIG. 18, the content of G2F containing two galactoses at the non-reducing terminal was higher that of the antibodies contained in the F1 fraction and the F2 fraction in FIG. 20. That is, it was found that the antibody contained in the solution could be separated by the separation method of an embodiment of the present invention based on a difference in structure of sugar chain, specifically, based on a difference in the number of galactose residues at the non-reducing terminal.

In addition, it was shown that, comparing compositions of the antibodies contained in the F1 fraction and the F2 fraction in FIG. 20, the F1 fraction had a higher content of antibodies having structures of sugar chain containing no galactose at the non-reducing terminal. That is, it was found that the antibody contained in the solution could be separated by the separation method of an embodiment of the present invention based on the content of antibody having structures of sugar chain containing no galactose at the non-reducing terminal.

Since the antibody having structures of sugar chain containing no galactose at the non-reducing terminal had weak ADCC activity and CDC activity, the F1 fraction and the F2 fraction in FIG. 20 and the F2 fraction in FIG. 18 were considered to be suitable for treatment of autoimmune diseases and the like. Since the antibody having structures of sugar chain containing galactose at the non-reducing terminal had strong ADCC activity and CDC activity, the F4 fraction in FIG. 20 was considered to be suitable for treatment of cancer and the like.

[Table 2]

| Name of structure of sugar chain | Proportion of structure of sugar chain [peak area proportion of each structure of sugar chain] | | | |
|---|---|---|---|---|
| | F1 fraction in FIG. 20 | F2 fraction in FIG. 20 | F2 fraction in FIG. 18 | F4 fraction in FIG. 20 |
| Man5 G1F-GN | 8.2 | 2.1 | - | - |
| G0F-GN | 2.1 | - | - | - |
| G0F | 54.4 | 53.4 | 37.5 | 7.6 |
| GIFa | 9.5 | 18.1 | 33.3 | 59.9 |
| G1Fb | 12.4 | 13.1 | 11.2 | 3.2 |
| G2F | 3.0 | 5.6 | 10.5 | 22.2 |
| G2F+SA | - | - | - | 1.6 |
| G2F+SA2 | - | - | 1.2 | - |
| Other | 10.3 | 7.7 | 6.3 | 5.6 |

[Table 3]

| Name of sugar chain structure | Sugar chain structure |
|---|---|
| G0F-GN | 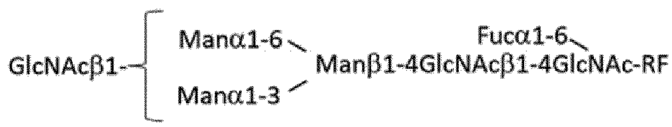 |
| G0 | 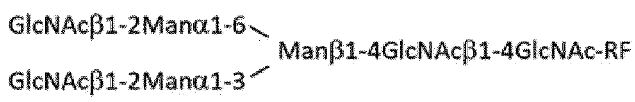 |
| G0F | 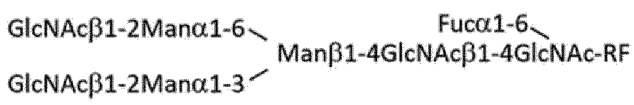 |
| G1F-GN | 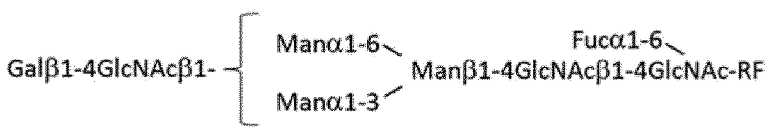 |

(continued)

| Name of sugar chain structure | Sugar chain structure |
|---|---|
| Man5 | 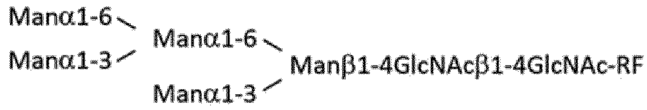 |
| G1 | 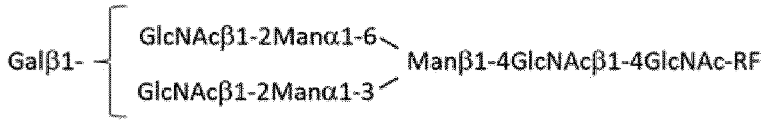 |
| G1Fa | 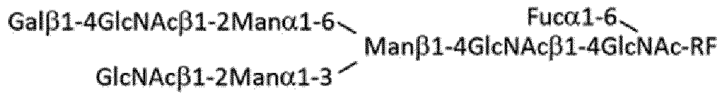 |
| G1Fb | 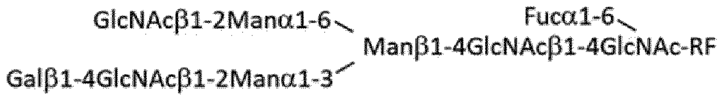 |
| G2F | 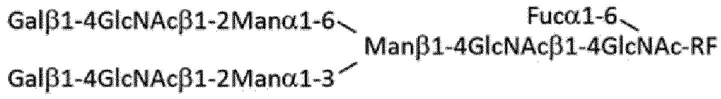 |
| G2F+SA | 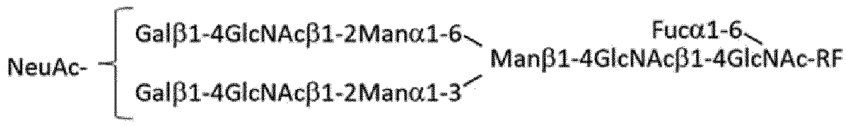 |
| G2F+SA2 | 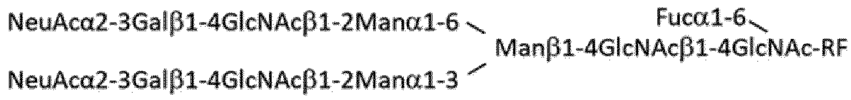 |

The abbreviations used in Table 3 are as follows.

NeuAc: N-acetylneuraminic acid
Gal: galactose
GlcNAc: N-acetylglucosamine
Man: mannose
Fuc: fucose
RF: RapiFluor-MS fluorescent label

The present disclosure facilitates separation of a large amount of antibodies based on a difference in structure and activity of antibody drugs or antibody-related drugs, which has been difficult in the related art. In addition, the present disclosure makes it possible to improve the quality, which has been an issue when antibody drugs are produced, that is, reduce variations in lot-to-lot activity due to a difference in structure.

## Claims

**1.** A method for separating an antibody, comprising:

a step of applying a solution containing an antibody to a column filled with an antibody adsorption material containing an insoluble porous carrier and an Fc-binding protein immobilized on the carrier and adsorbing the antibody onto the antibody adsorption material; and
a step of applying a buffer solution to the column and eluting the antibody adsorbed onto the antibody adsorption material with a pH gradient,

wherein the pH gradient is a step gradient in which a pH of the buffer solution is lowered in steps, and wherein the magnitude of pH decrease is 0.1 or more and less than 1.

**2.** The method according to claim 1, wherein the insoluble porous carrier has a volume-based Mean particle Diameter of 30 to 150 μm.

**3.** The method according to claim 1, wherein the buffer solution is a solution containing a buffer substance that has a buffering capacity in a pH range of 3.0 or more and 6.0 or less and has a plurality of pKas within the range.

**4.** The method according to claim 1, wherein a ratio of a mass of the antibody to the volume of the antibody adsorption material is 1 mg/1.0 mL to 50 mg/0.1 mL.

**5.** The method according to any one of claims 1 to 4, wherein the Fc-binding protein is human FcγRIIIa.

**6.** The method according to claim 5, wherein the method is based on a difference in a structure of sugar chain of the antibody.

**7.** The method according to claim 6, wherein the difference in the structure of sugar chain is a difference in the number of galactose residues at the non-reducing terminal in the structure of sugar chain.

**8.** The method according to claim 5, wherein the human FcγRIIIa is a polypeptide shown in the following (1), (2) or (3):

(1) a polypeptide including the amino acid sequence from the 17th glycine residue (Gly) to the 192nd glutamine residue (Gln) in the amino acid sequence set forth as SEQ ID NO: 1,
(2) a polypeptide having one or more amino acid residue substitutions, deletions, insertions or additions in the amino acid sequence from the 17th glycine residue (Gly) to the 192nd glutamine residue (Gln) in the amino acid sequence set forth as SEQ ID NO: 1, and having binding activity to the antibody, and
(3) a polypeptide having a homology of 70% or more with the amino acid sequence from the 17th glycine residue (Gly) to the 192nd glutamine residue (Gln) in the amino acid sequence set forth as SEQ ID NO: 1 and having binding activity to the antibody.

**9.** An antibody composition containing 15 mass% or more of an antibody which is eluted in the following citrate buffer solution with a pH of 4.0 or less when a monoclonal antibody solution is fractionated under the following measurement conditions, based on a total mass of the antibody composition:
(Measurement conditions)

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.07 mL/min
Solvent: phosphate buffered saline (pH 7.4)
Step gradient: add 3.73 mL each of a 50 mM citrate buffer solution (pH 6.0), a 50 mM citrate buffer solution (pH 5.5), a 50 mM citrate buffer solution (pH 5.0), a 50 mM citrate buffer solution (pH 4.8), a 50 mM citrate buffer solution (pH 4.6), a 50 mM citrate buffer solution (pH 4.4), a 50 mM citrate buffer solution (pH 4.2), a 50 mM citrate buffer solution (pH 4.0), a 50 mM citrate buffer solution (pH 3.8), a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)
Injection amount: 2,000 μL (antibody amount: 5 mg)

**10.** An antibody composition containing 15 mass% or more of an antibody which is eluted in the following citrate buffer solution with a pH of 4.2 or more when a monoclonal antibody solution is fractionated under the following measurement conditions, based on a total mass of the antibody composition:
(Measurement conditions)

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.07 mL/min
Solvent: phosphate buffered saline (pH 7.4)
Step gradient: add 3.73 mL each of a 50 mM citrate buffer solution (pH 6.0), a 50 mM citrate buffer solution (pH 5.5), a 50 mM citrate buffer solution (pH 5.0), a 50 mM citrate buffer solution (pH 4.8), a 50 mM citrate buffer solution (pH 4.6), a 50 mM citrate buffer solution (pH 4.4), a 50 mM citrate buffer solution (pH 4.2), a 50 mM citrate buffer solution (pH 4.0), a 50 mM citrate buffer solution (pH 3.8), a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)
Injection amount: 2,000 μL (antibody amount: 5 mg)

**11.** A concentrate of an antibody solution,

wherein the antibody solution and the concentrate contain the antibody which is eluted in the following citrate buffer solution with a pH of 4.0 or less when a monoclonal antibody solution is fractionated under the following measurement conditions, and
wherein a content of the antibody based on a total amount of the concentrate is at least twice a content of the antibody based on a total amount of the antibody solution:
(Measurement conditions)

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.07 mL/min
Solvent: phosphate buffered saline (pH 7.4) Step gradient: add 3.73 mL each of a 50 mM citrate buffer solution (pH 6.0), a 50 mM citrate buffer solution (pH 5.5), a 50 mM citrate buffer solution (pH 5.0), a 50 mM citrate buffer solution (pH 4.8), a 50 mM citrate buffer solution (pH 4.6), a 50 mM citrate buffer solution (pH 4.4), a 50 mM citrate buffer solution (pH 4.2), a 50 mM citrate buffer solution (pH 4.0), a 50 mM citrate buffer solution (pH 3.8), a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)
Injection amount: 2,000 μL (antibody amount: 5 mg)

**12.** A concentrate of an antibody solution,

wherein the antibody solution and the concentrate contain the antibody which is eluted in the following citrate buffer solution with a pH of 4.2 or more when a monoclonal antibody solution is fractionated under the following measurement conditions, and
wherein a content of the antibody based on a total amount of the concentrate is at least twice a content of the antibody based on a total amount of the antibody solution:
(Measurement conditions)

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.07 mL/min
Solvent: phosphate buffered saline (pH 7.4)
Step gradient: add 3.73 mL each of a 50 mM citrate buffer solution (pH 6.0), a 50 mM citrate buffer solution (pH 5.5), a 50 mM citrate buffer solution (pH 5.0), a 50 mM citrate buffer solution (pH 4.8), a 50 mM citrate buffer solution (pH 4.6), a 50 mM citrate buffer solution (pH 4.4), a 50 mM citrate buffer solution (pH 4.2), a 50 mM citrate buffer solution (pH 4.0), a 50 mM citrate buffer solution (pH 3.8), a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)
Injection amount: 2,000 μL (antibody amount: 5 mg)

**13.** An antibody composition containing 15 mass% or more of an antibody which is eluted in the following citrate buffer solution with a pH of 4.0 or less when a monoclonal antibody solution is fractionated under the following measurement conditions, based on a total mass of the antibody composition:
(Measurement conditions)

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.20 mL/min
Solvent: phosphate buffered saline (pH 7.4)
Step gradient: add 9.82 mL each of a 50 mM citrate buffer solution (pH 6.0), a 50 mM citrate buffer solution (pH 5.5), a 50 mM citrate buffer solution (pH 5.0), and a 50 mM citrate buffer solution (pH 4.8) in that order, add 29.46 mL each of a 50 mM citrate buffer solution (pH 4.6), a 50 mM citrate buffer solution (pH 4.4), a 50 mM citrate buffer solution (pH 4.2), a 50 mM citrate buffer solution (pH 4.0), and a 50 mM citrate buffer solution (pH 3.8) in that order, and add 9.82 mL each of a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)
Injection amount: 1000-8000 μL (antibody amount: 5-40 mg)

**14.** An antibody composition containing 15 mass% or more of an antibody which is eluted in the following citrate buffer solution with a pH of 4.2 or more when a monoclonal antibody solution is fractionated under the following measurement conditions, based on a total mass of the antibody composition:
(Measurement conditions)

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.20 mL/min
Solvent: phosphate buffered saline (pH 7.4)
Step gradient: add 9.82 mL each of a 50 mM citrate buffer solution (pH 6.0), a 50 mM citrate buffer solution (pH 5.5), a 50 mM citrate buffer solution (pH 5.0), and a 50 mM citrate buffer solution (pH 4.8) in that order, add 29.46 mL each of a 50 mM citrate buffer solution (pH 4.6), a 50 mM citrate buffer solution (pH 4.4), a 50 mM citrate buffer solution (pH 4.2), a 50 mM citrate buffer solution (pH 4.0), and a 50 mM citrate buffer solution (pH 3.8) in that order, and add 9.82 mL each of a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution

Column temperature: 25°C
Detection: UV (280 nm)
Injection amount: 1000-8000 μL (antibody amount: 5-40 mg)

**15.** A concentrate of an antibody solution,

wherein the antibody solution and the concentrate contain the antibody which is eluted in the following citrate buffer solution with a pH of 4.0 or less when a monoclonal antibody solution is fractionated under the following measurement conditions, and
wherein a content of the antibody based on a total amount of the concentrate is at least twice a content of the antibody based on a total amount of the antibody solution:
(Measurement conditions)

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.20 mL/min
Solvent: phosphate buffered saline (pH 7.4)
Step gradient: add 9.82 mL each of a 50 mM citrate buffer solution (pH 6.0), a 50 mM citrate buffer solution (pH 5.5), a 50 mM citrate buffer solution (pH 5.0), and a 50 mM citrate buffer solution (pH 4.8) in that order, add 29.46 mL each of a 50 mM citrate buffer solution (pH 4.6), a 50 mM citrate buffer solution (pH 4.4), a 50 mM citrate buffer solution (pH 4.2), a 50 mM citrate buffer solution (pH 4.0), and a 50 mM citrate buffer solution (pH 3.8) in that order, and add 9.82 mL each of a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)
Injection amount: 1000-8000 μL (antibody amount: 5-40 mg)

**16.** A concentrate of an antibody solution,

wherein the antibody solution and the concentrate contain the antibody which is eluted in the following citrate buffer solution with a pH of 4.2 or more when a monoclonal antibody solution is fractionated under the following measurement conditions, and
wherein a content of the antibody based on a total amount of the concentrate is at least twice a content of the antibody based on a total amount of the antibody solution:
(Measurement conditions)

Column: Tricorn 5/50 (φ5.0 mm×50 mm, commercially available from Cytiva)
Adsorption material: FcR36i_Cys immobilized gel obtained by reacting 30 mg of Fc-binding protein FcR36i_Cys (SEQ ID NO: 2) with 1 g of a porous hydrophilic vinyl polymer for an adsorption material (TOYOPEARL (filler for liquid chromatography, commercially available from Tosoh Corporation), volume-based Mean particle Diameter: 45 μm)
Flow rate: 0.20 mL/min
Solvent: phosphate buffered saline (pH 7.4)
Step gradient: add 9.82 mL each of a 50 mM citrate buffer solution (pH 6.0), a 50 mM citrate buffer solution (pH 5.5), a 50 mM citrate buffer solution (pH 5.0), and a 50 mM citrate buffer solution (pH 4.8) in that order, add 29.46 mL each of a 50 mM citrate buffer solution (pH 4.6), a 50 mM citrate buffer solution (pH 4.4), a 50 mM citrate buffer solution (pH 4.2), a 50 mM citrate buffer solution (pH 4.0), and a 50 mM citrate buffer solution (pH 3.8) in that order, and add 9.82 mL each of a 50 mM citrate buffer solution (pH 3.6) and a 50 mM citrate buffer solution (pH 3.4) in that order
50 mM citrate buffer solution: a mixed solution containing a 50 mM citrate aqueous solution and a 50 mM trisodium citrate aqueous solution
Column temperature: 25°C
Detection: UV (280 nm)

Injection amount: 1000-8000 μL (antibody amount: 5-40 mg)

***Fig.1***

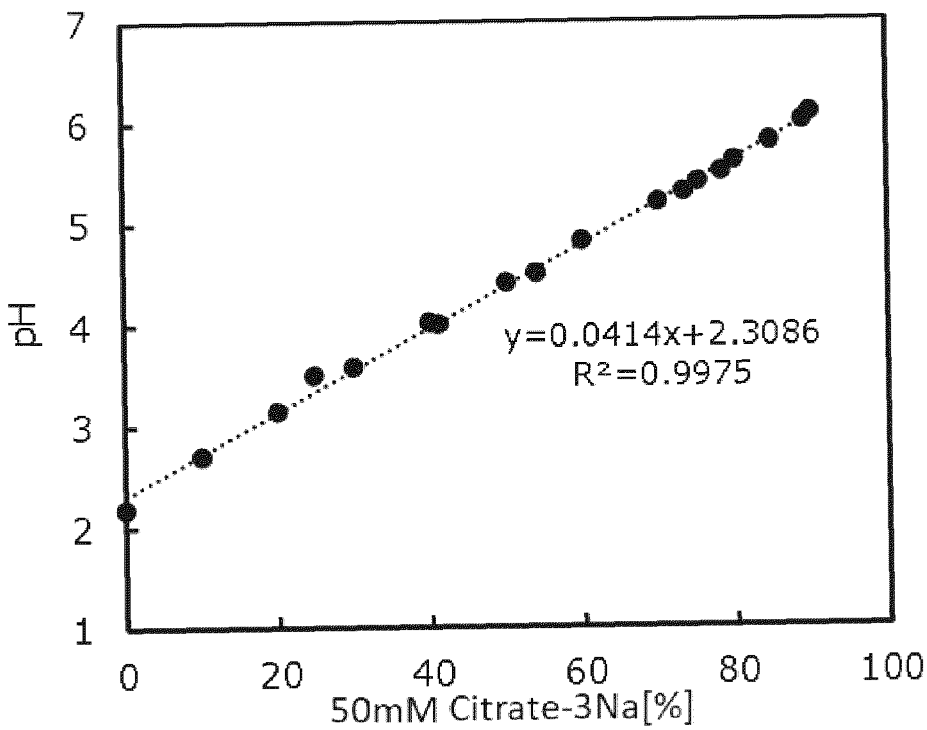

7
6
5
4
3
2
1
pH
0
20
40
60
80
100
50mM Citrate-3Na[%]
y=0.0414x+2.3086
R²=0.9975

*Fig.2*

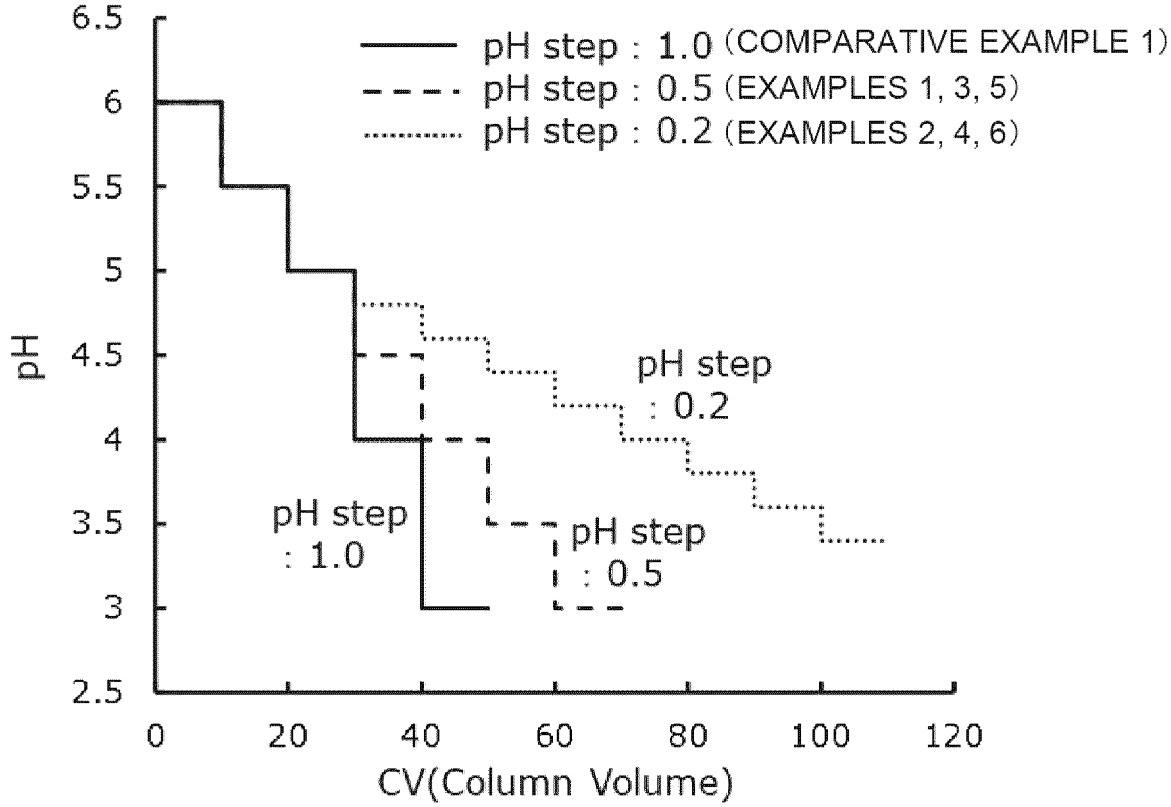
pH step : 1.0 (COMPARATIVE EXAMPLE 1)
pH step : 0.5 (EXAMPLES 1, 3, 5)
pH step : 0.2 (EXAMPLES 2, 4, 6)
pH
6.5
6
5.5
5
4.5
4
3.5
3
2.5
pH step : 0.2
pH step : 1.0
pH step : 0.5
0
20
40
60
80
100
120
CV(Column Volume)

*Fig.3*

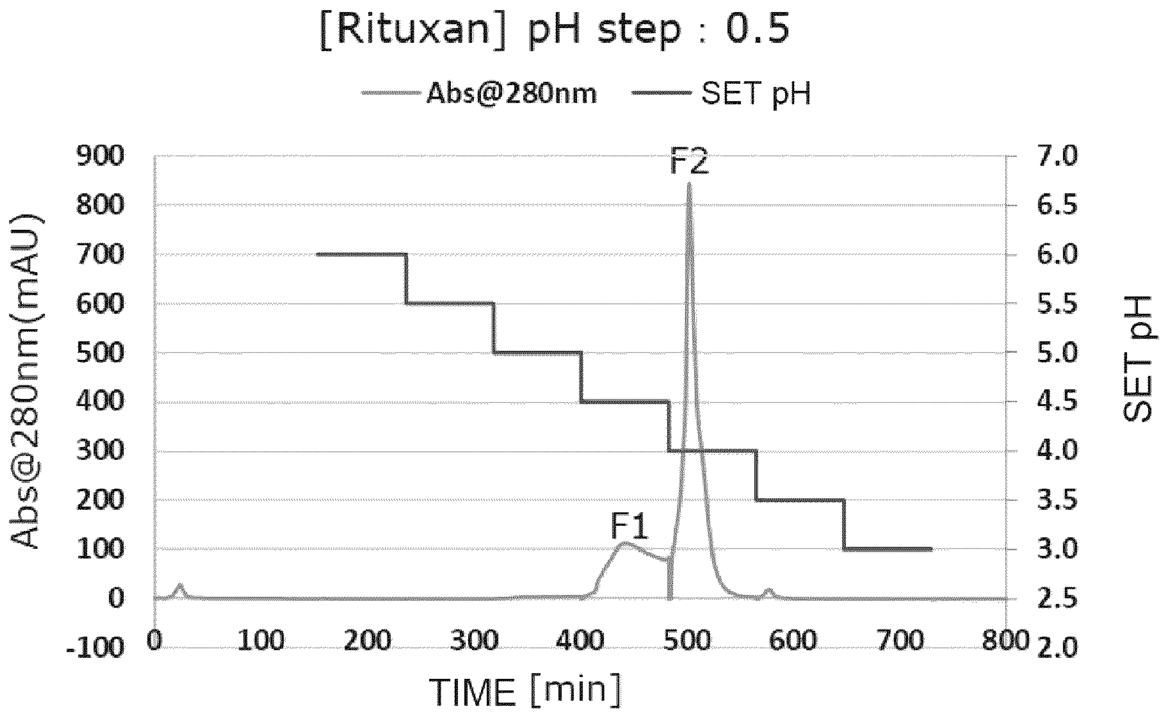
[Rituxan] pH step : 0.5
Abs@280nm
SET pH
F1
F2
Abs@280nm(mAU)
SET pH
TIME [min]
900
800
700
600
500
400
300
200
100
0
-100
7.0
6.5
6.0
5.5
5.0
4.5
4.0
3.5
3.0
2.5
2.0
0
100
200
300
400
500
600
700
800

# Fig.4

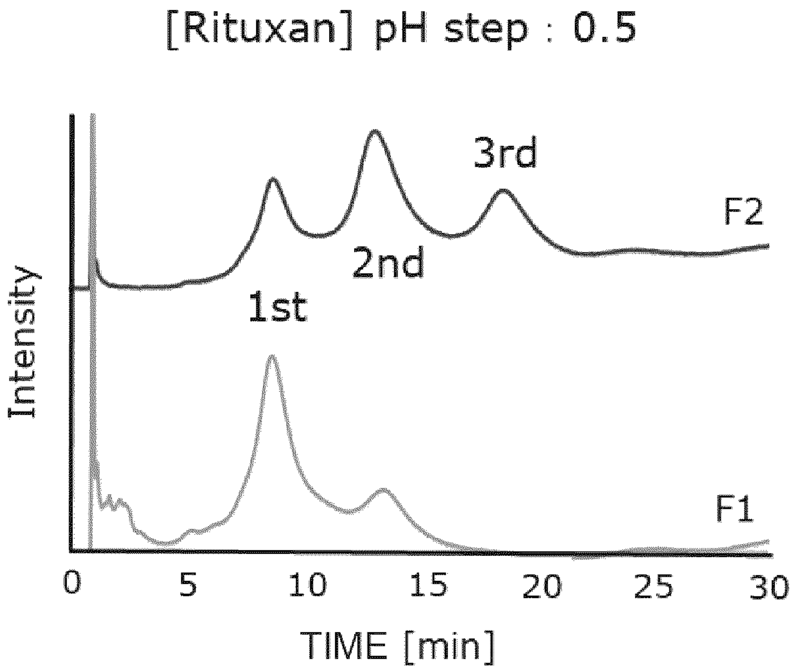
[Rituxan] pH step : 0.5
3rd
F2
2nd
1st
Intensity
F1
0
5
10
15
20
25
30
TIME [min]

*Fig.5*

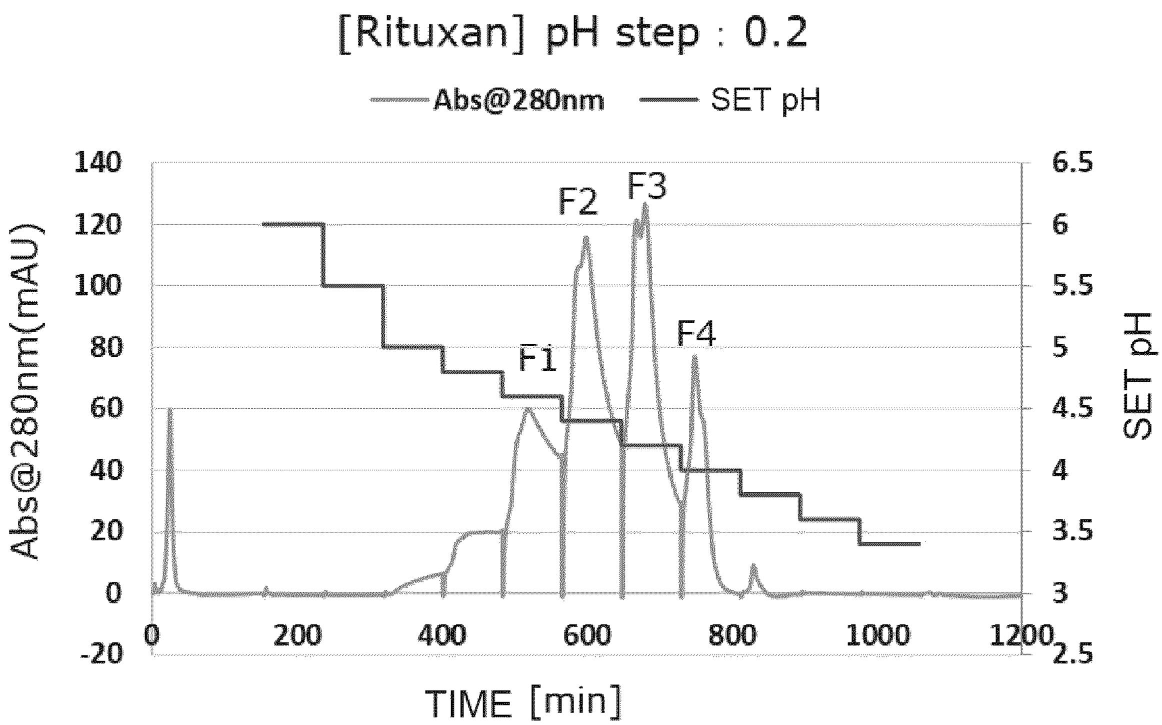
[Rituxan] pH step : 0.2
Abs@280nm
SET pH
Abs@280nm(mAU)
SET pH
TIME [min]
F1
F2
F3
F4
140
120
100
80
60
40
20
0
-20
6.5
6
5.5
5
4.5
4
3.5
3
2.5
0
200
400
600
800
1000
1200

# Fig.6

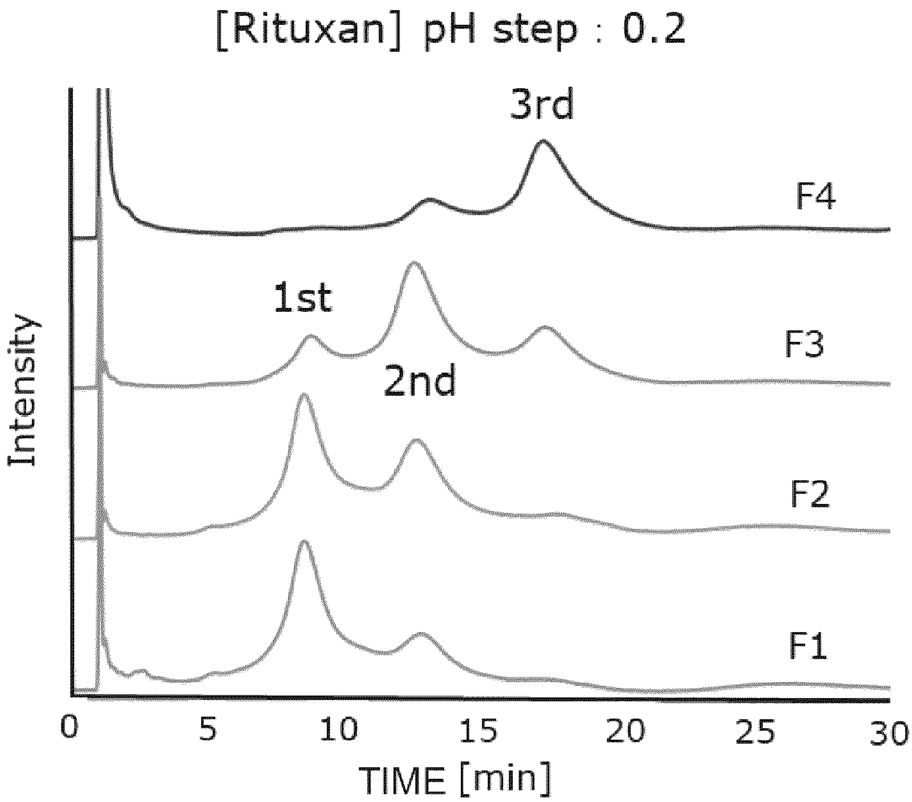
[Rituxan] pH step : 0.2
3rd
F4
1st
F3
2nd
F2
F1
Intensity
0
5
10
15
20
25
30
TIME [min]

*Fig.7*

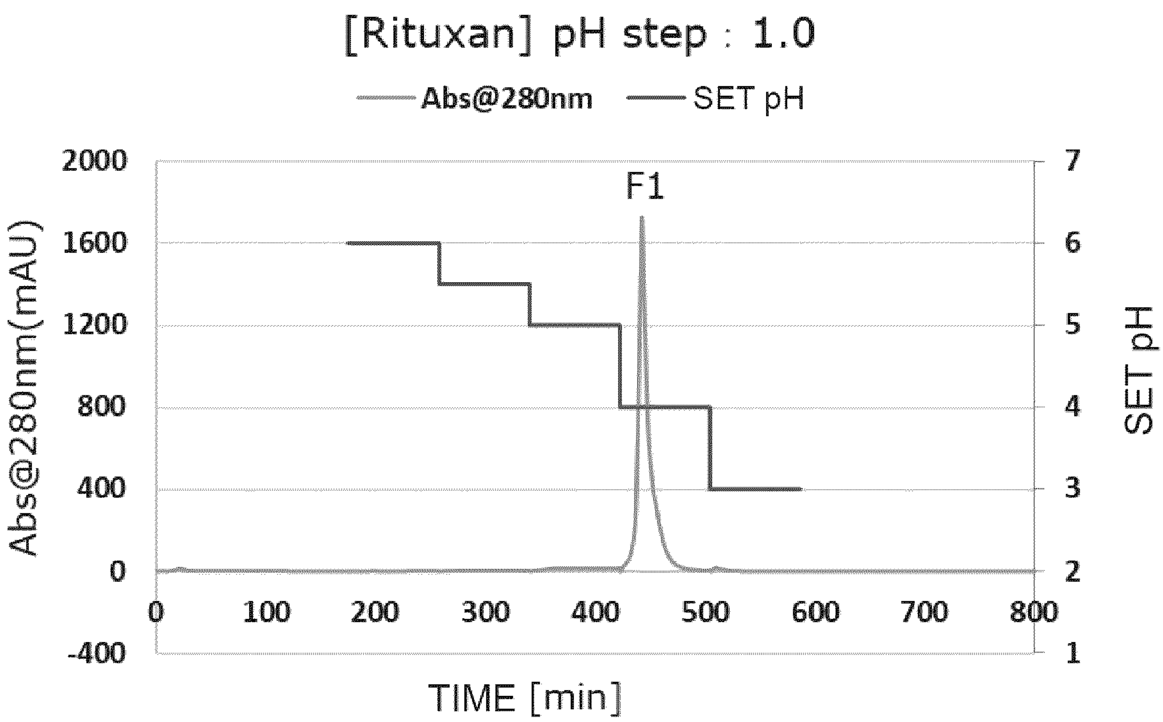
[Rituxan] pH step : 1.0
Abs@280nm
SET pH
F1
Abs@280nm(mAU)
SET pH
TIME [min]
2000
1600
1200
800
400
0
-400
7
6
5
4
3
2
1
0
100
200
300
400
500
600
700
800

*Fig.8*

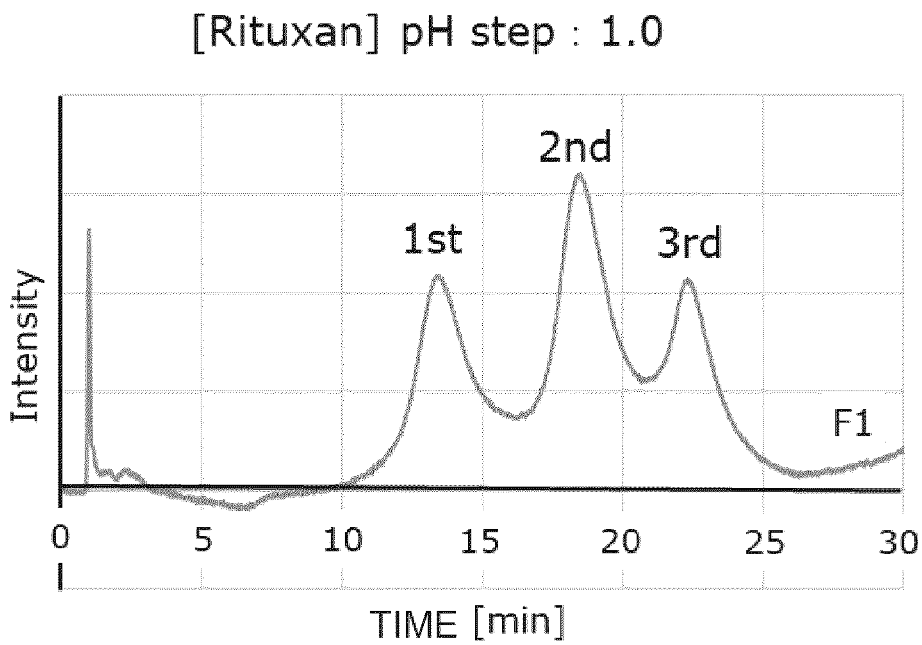
[Rituxan] pH step : 1.0
Intensity
1st
2nd
3rd
F1
0
5
10
15
20
25
30
TIME [min]

*Fig.9*

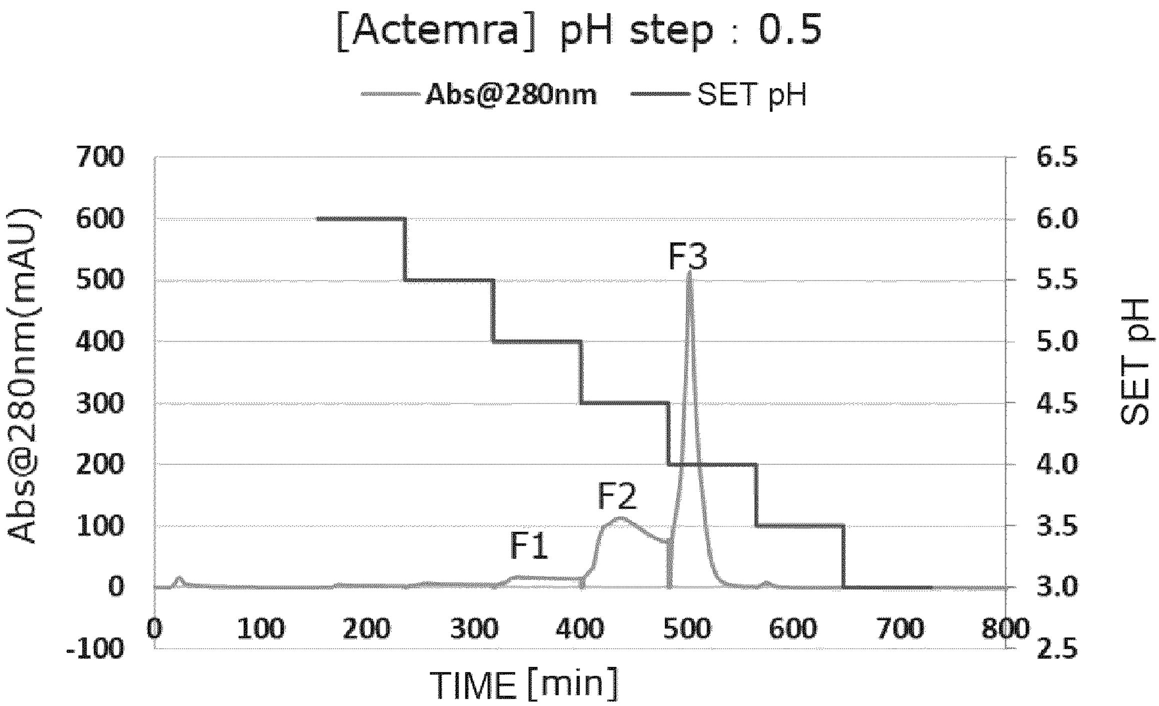
[Actemra] pH step : 0.5
Abs@280nm
SET pH
Abs@280nm(mAU)
SET pH
TIME [min]
F1
F2
F3
700
600
500
400
300
200
100
0
-100
6.5
6.0
5.5
5.0
4.5
4.0
3.5
3.0
2.5
0
100
200
300
400
500
600
700
800

# Fig.10

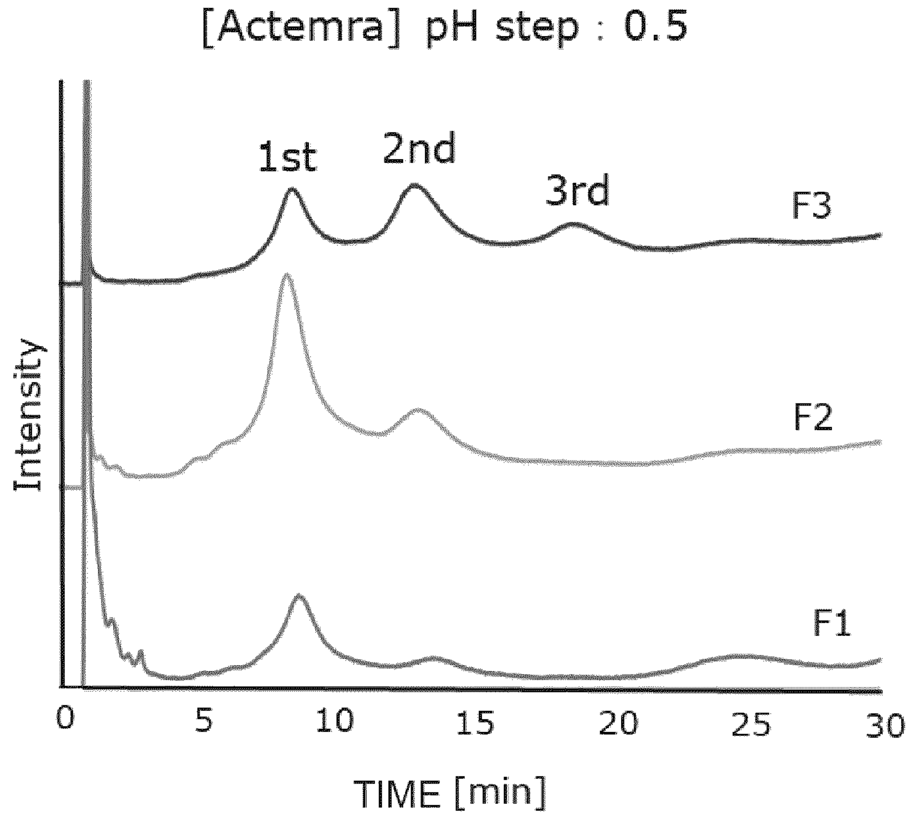
[Actemra] pH step : 0.5
1st
2nd
3rd
F3
F2
F1
Intensity
0
5
10
15
20
25
30
TIME [min]

*Fig.11*

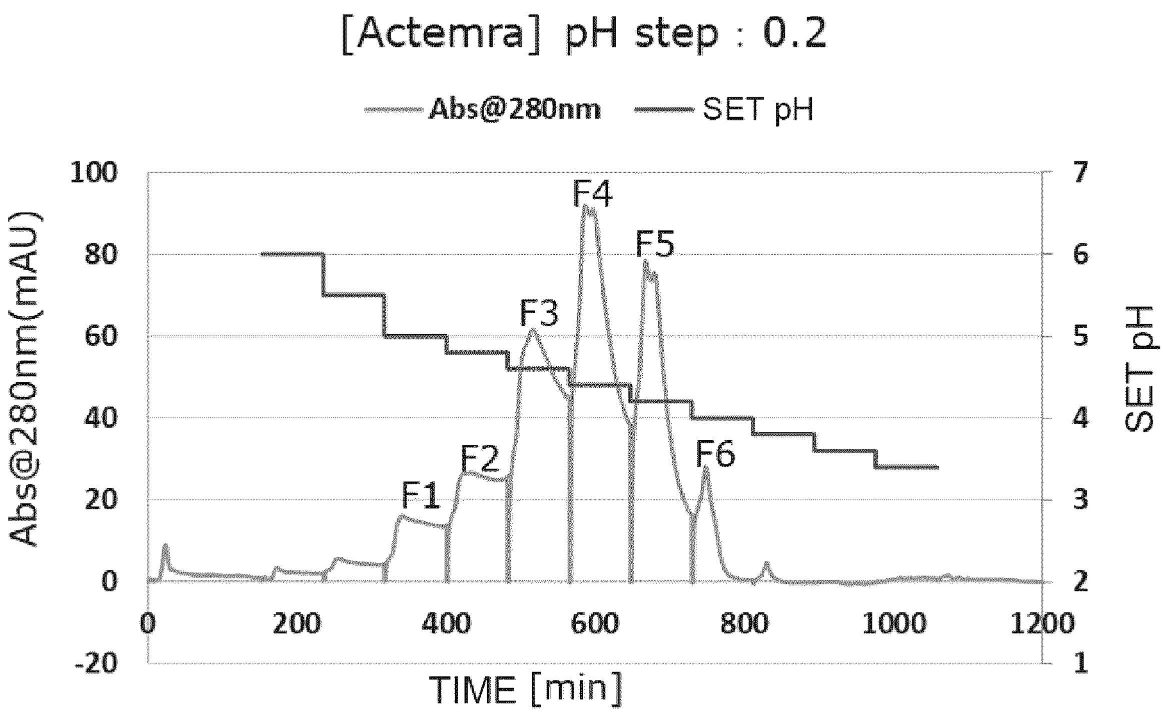
[Actemra] pH step : 0.2
Abs@280nm
SET pH
Abs@280nm(mAU)
100
80
60
40
20
0
-20
0
200
400
600
800
1000
1200
TIME [min]
7
6
5
4
3
2
1
SET pH
F1
F2
F3
F4
F5
F6

# Fig.12

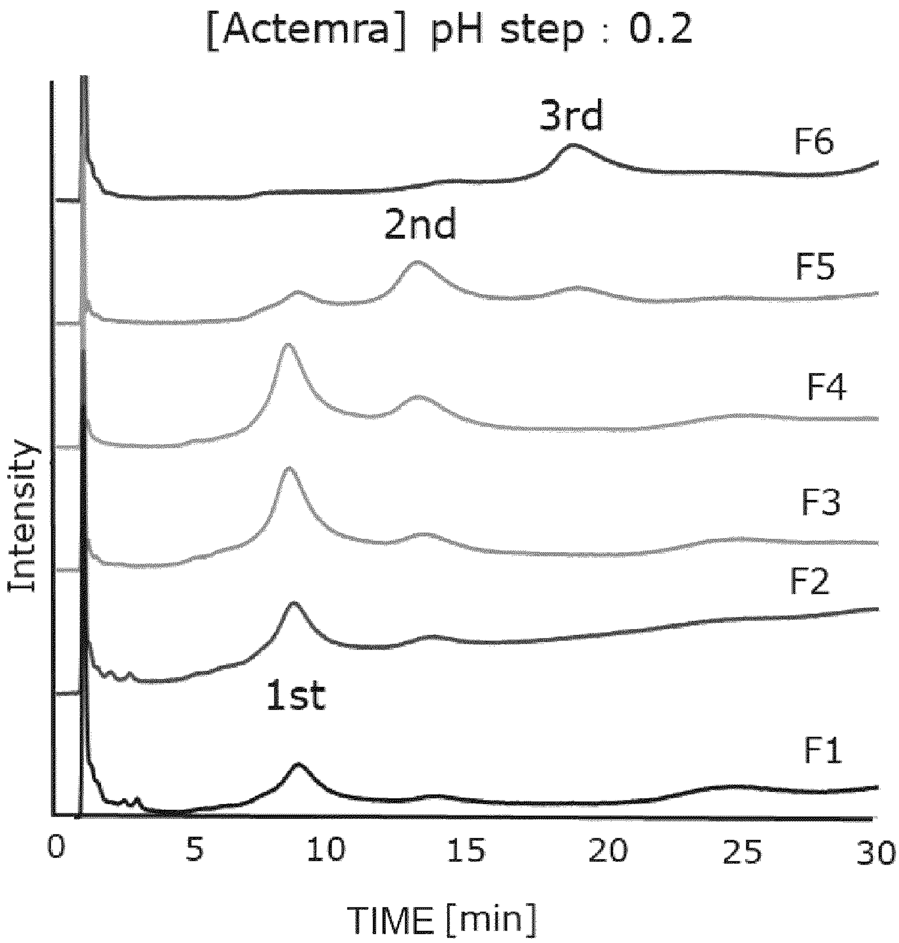
[Actemra] pH step : 0.2
3rd
F6
2nd
F5
F4
F3
F2
1st
F1
Intensity
0
5
10
15
20
25
30
TIME [min]

# Fig.13

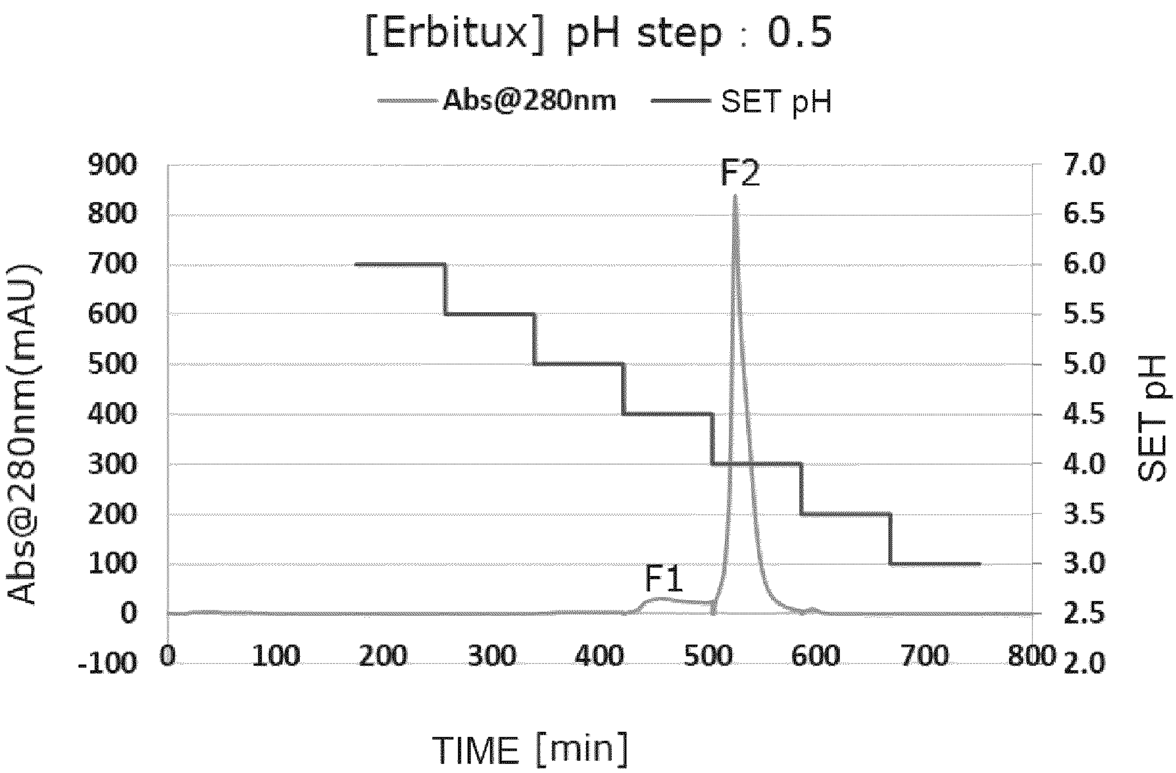
[Erbitux] pH step : 0.5
Abs@280nm
SET pH
Abs@280nm(mAU)
SET pH
TIME [min]
F1
F2
900
800
700
600
500
400
300
200
100
0
-100
0
100
200
300
400
500
600
700
800
7.0
6.5
6.0
5.5
5.0
4.5
4.0
3.5
3.0
2.5
2.0

*Fig.14*

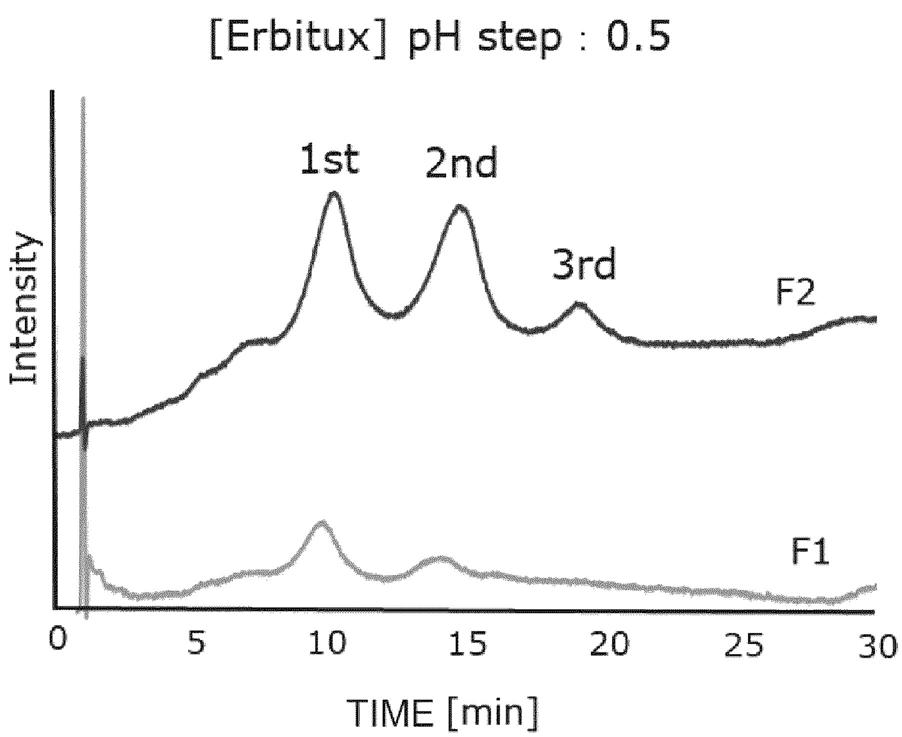
[Erbitux] pH step : 0.5
1st
2nd
3rd
F2
F1
Intensity
0
5
10
15
20
25
30
TIME [min]

# Fig.15

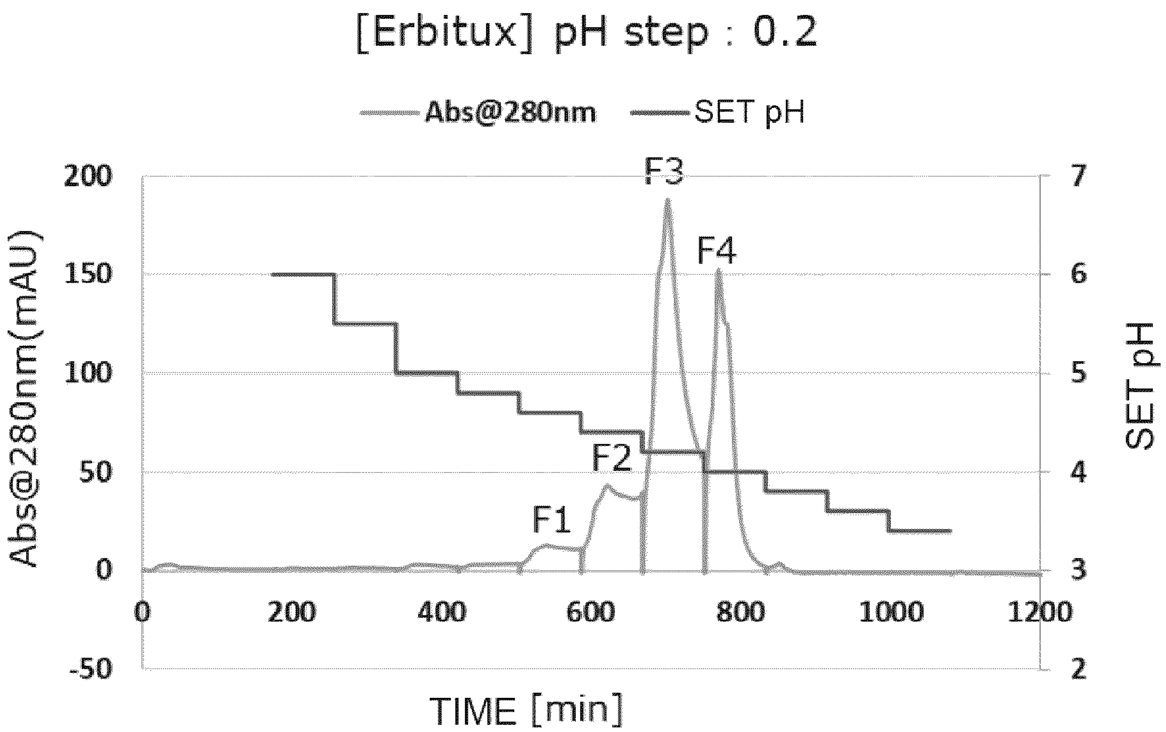
[Erbitux] pH step : 0.2
Abs@280nm
SET pH
Abs@280nm(mAU)
SET pH
TIME [min]
F1
F2
F3
F4
200
150
100
50
0
-50
7
6
5
4
3
2
0
200
400
600
800
1000
1200

# Fig.16

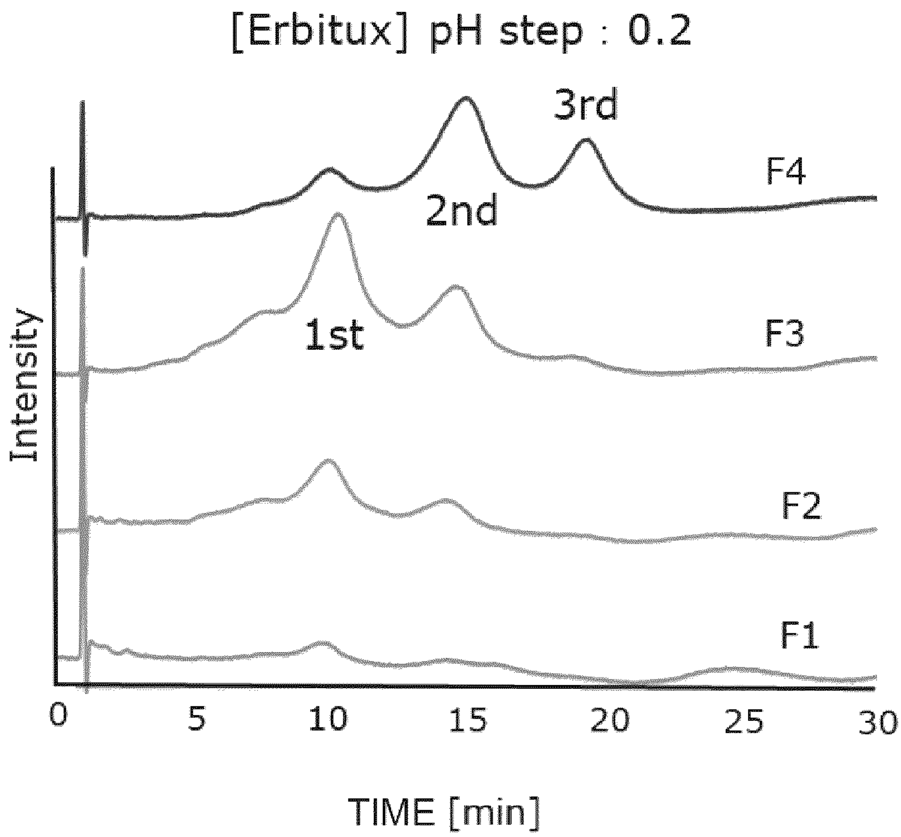
[Erbitux] pH step : 0.2
3rd
2nd
1st
F4
F3
F2
F1
Intensity
0
5
10
15
20
25
30
TIME [min]

# Fig.17

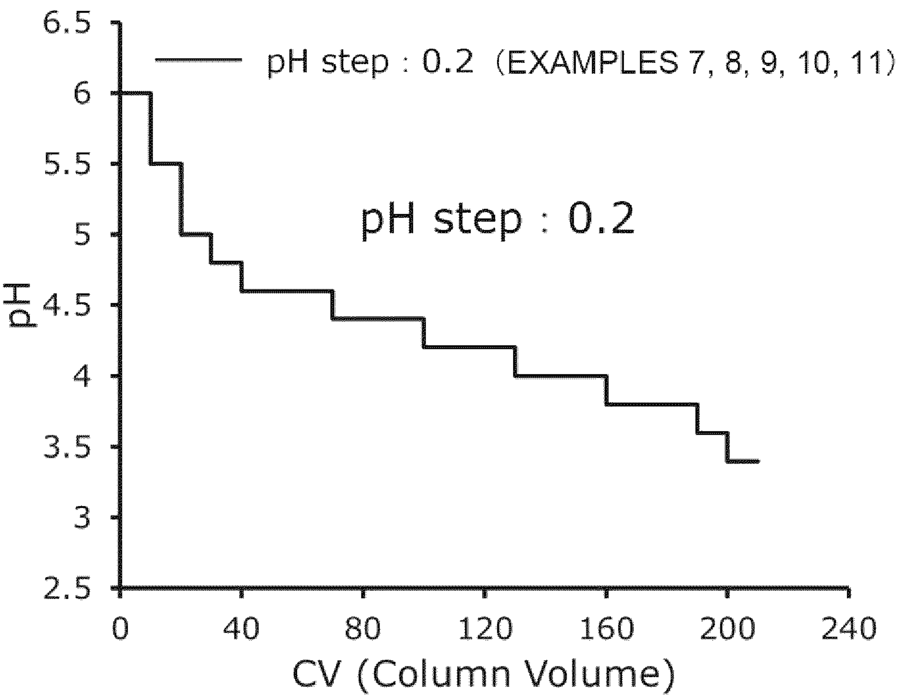
pH step : 0.2 (EXAMPLES 7, 8, 9, 10, 11)
pH step : 0.2
pH
6.5
6
5.5
5
4.5
4
3.5
3
2.5
0
40
80
120
160
200
240
CV (Column Volume)

*Fig.18*

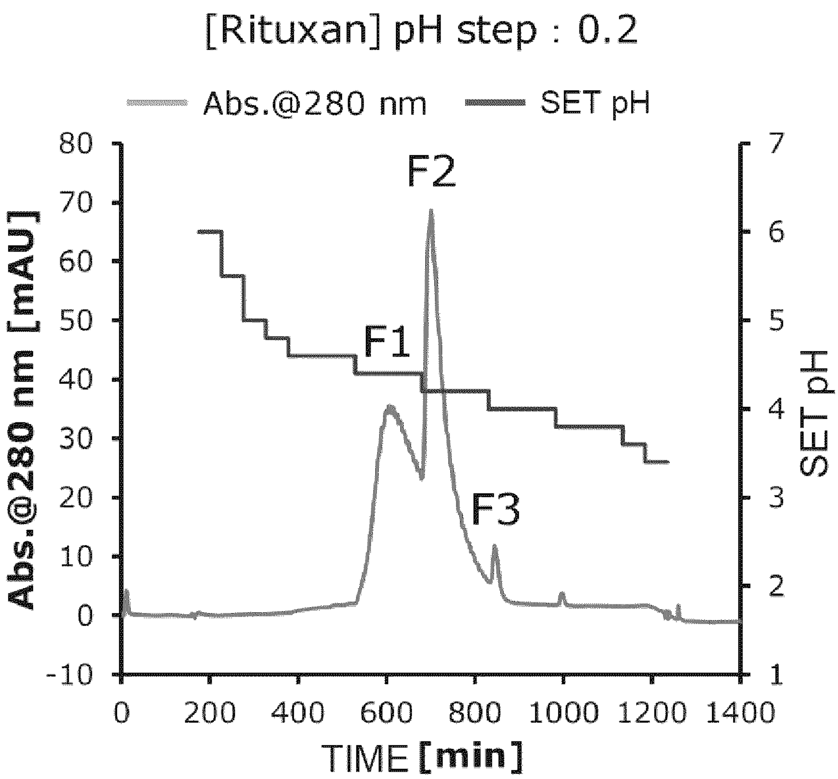
[Rituxan] pH step : 0.2
Abs.@280 nm
SET pH
80
70
60
50
40
30
20
10
0
-10
Abs.@280 nm [mAU]
7
6
5
4
3
2
1
SET pH
F1
F2
F3
0
200
400
600
800
1000
1200
1400
TIME [min]

*Fig.19*

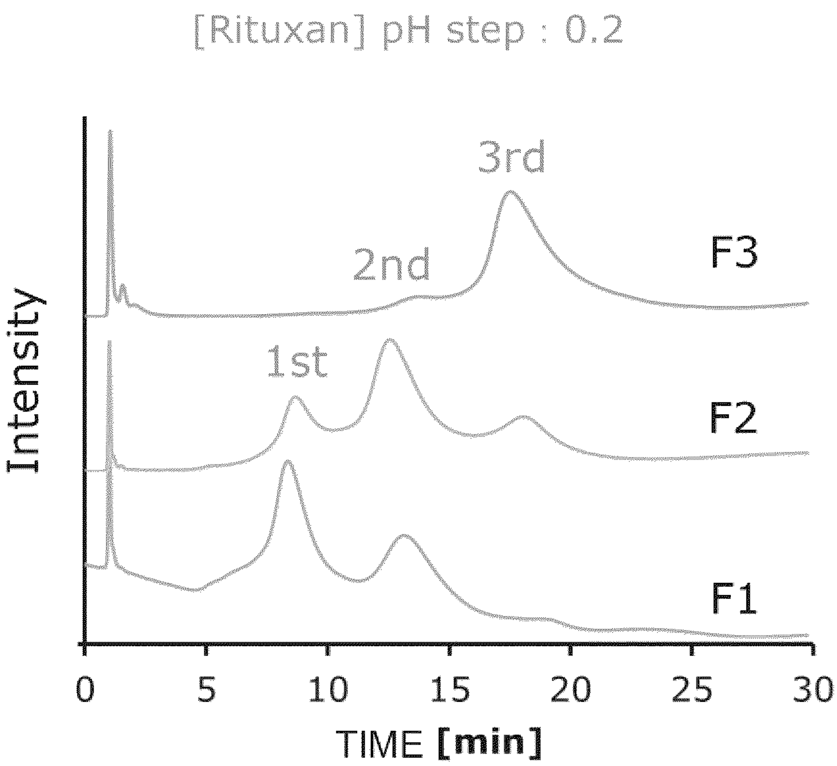
[Rituxan] pH step : 0.2
3rd
2nd
1st
F3
F2
F1
Intensity
0
5
10
15
20
25
30
TIME [min]

*Fig.20*

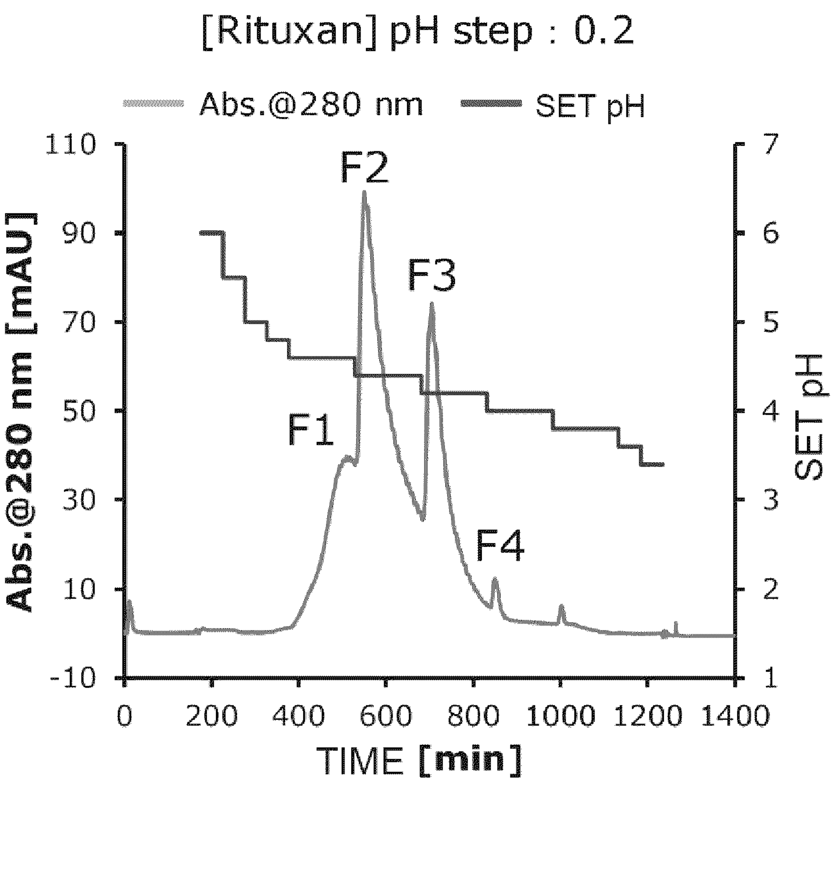
[Rituxan] pH step : 0.2
Abs.@280 nm
SET pH
Abs.@280 nm [mAU]
SET pH
TIME [min]
F1
F2
F3
F4
110
90
70
50
30
10
-10
7
6
5
4
3
2
1
0
200
400
600
800
1000
1200
1400

***Fig.21***

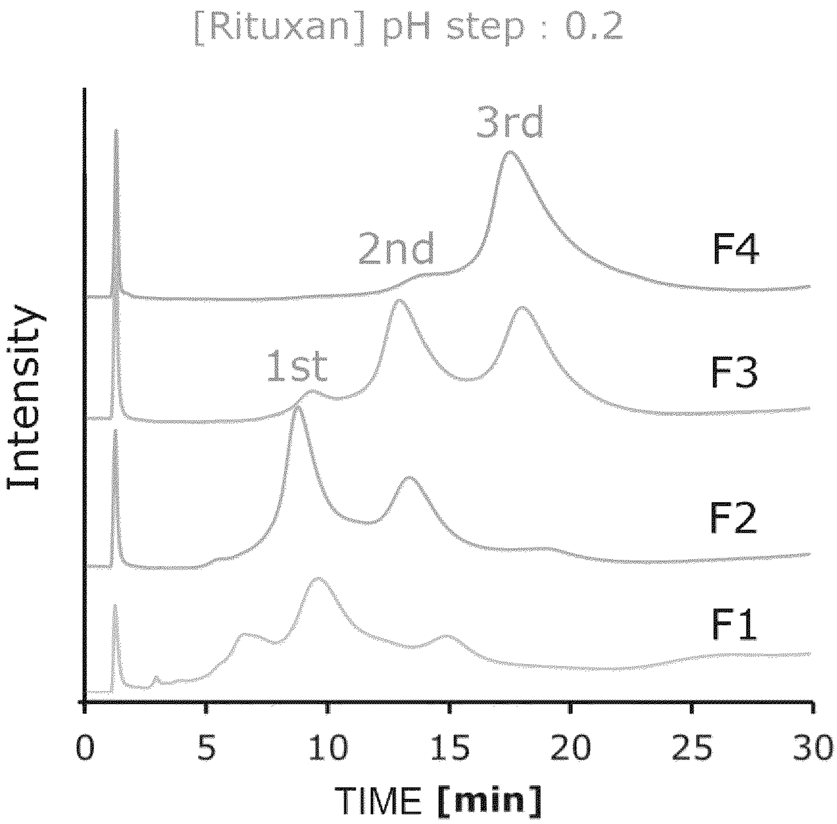
[Rituxan] pH step : 0.2
3rd
2nd
1st
F4
F3
F2
F1
Intensity
0
5
10
15
20
25
30
TIME [min]

Fig.22

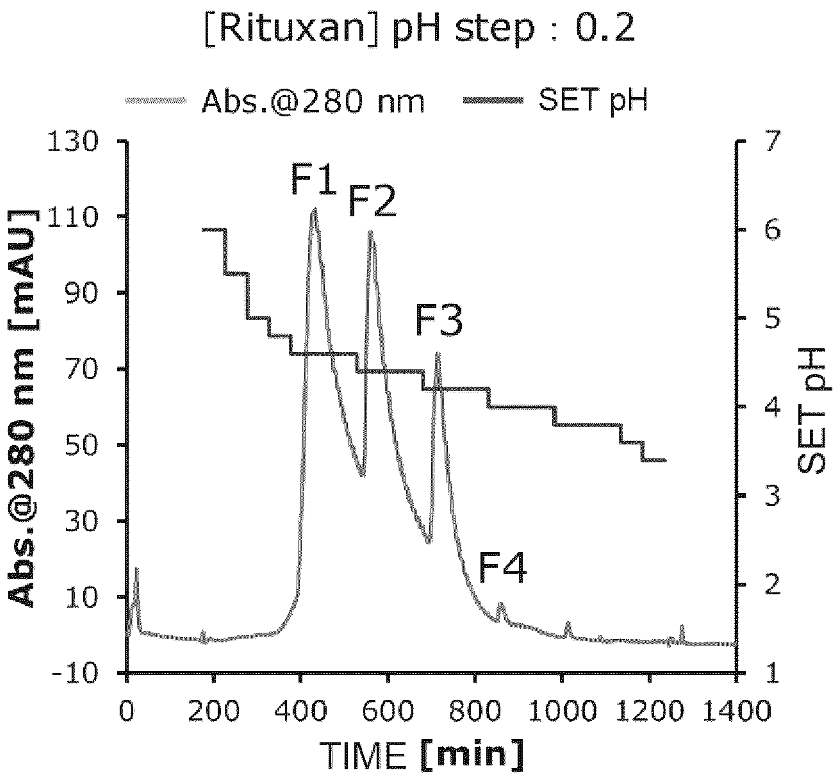
[Rituxan] pH step : 0.2
Abs.@280 nm
SET pH
F1
F2
F3
F4
Abs.@280 nm [mAU]
SET pH
TIME [min]
130
110
90
70
50
30
10
-10
7
6
5
4
3
2
1
0
200
400
600
800
1000
1200
1400

***Fig.23***

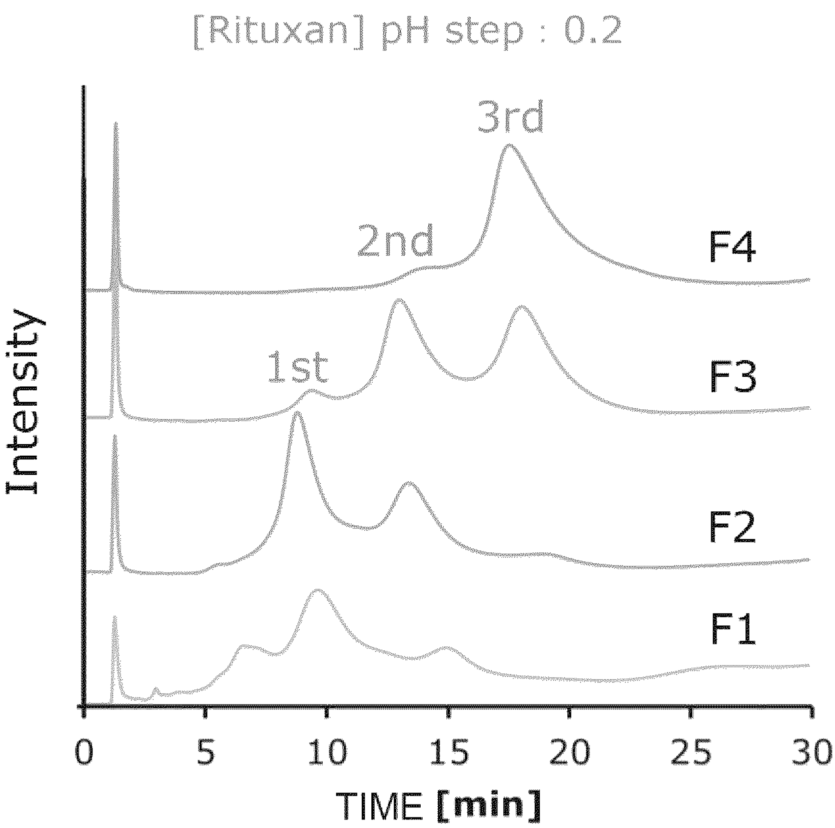
[Rituxan] pH step : 0.2
3rd
2nd
1st
F4
F3
F2
F1
Intensity
0
5
10
15
20
25
30
TIME [min]

# Fig.24

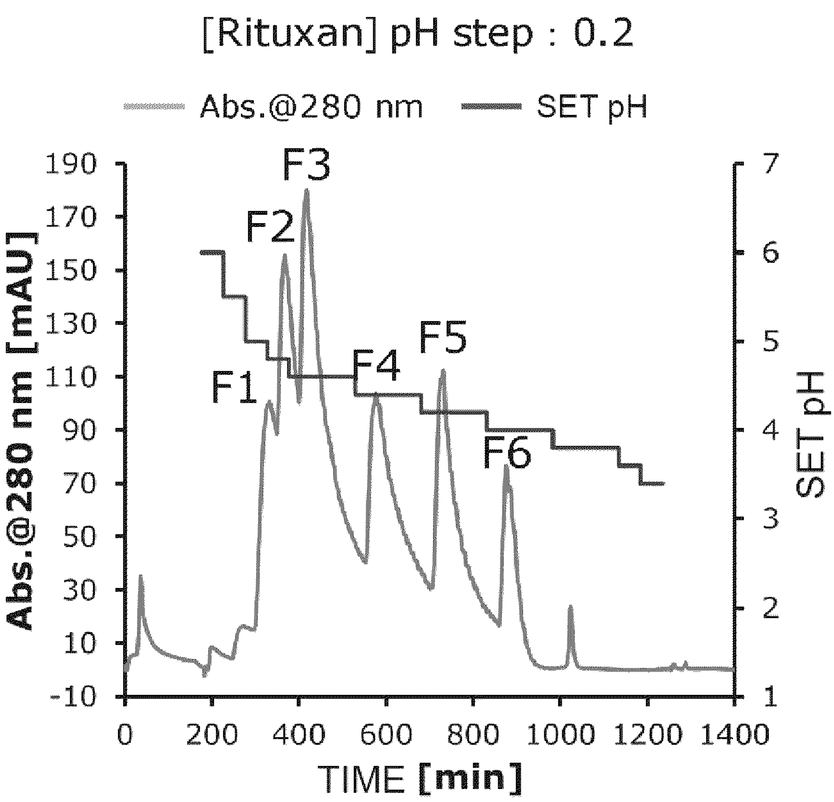
[Rituxan] pH step : 0.2
Abs.@280 nm
SET pH
F1
F2
F3
F4
F5
F6
Abs.@280 nm [mAU]
SET pH
TIME [min]
190
170
150
130
110
90
70
50
30
10
-10
7
6
5
4
3
2
1
0
200
400
600
800
1000
1200
1400

# Fig.25

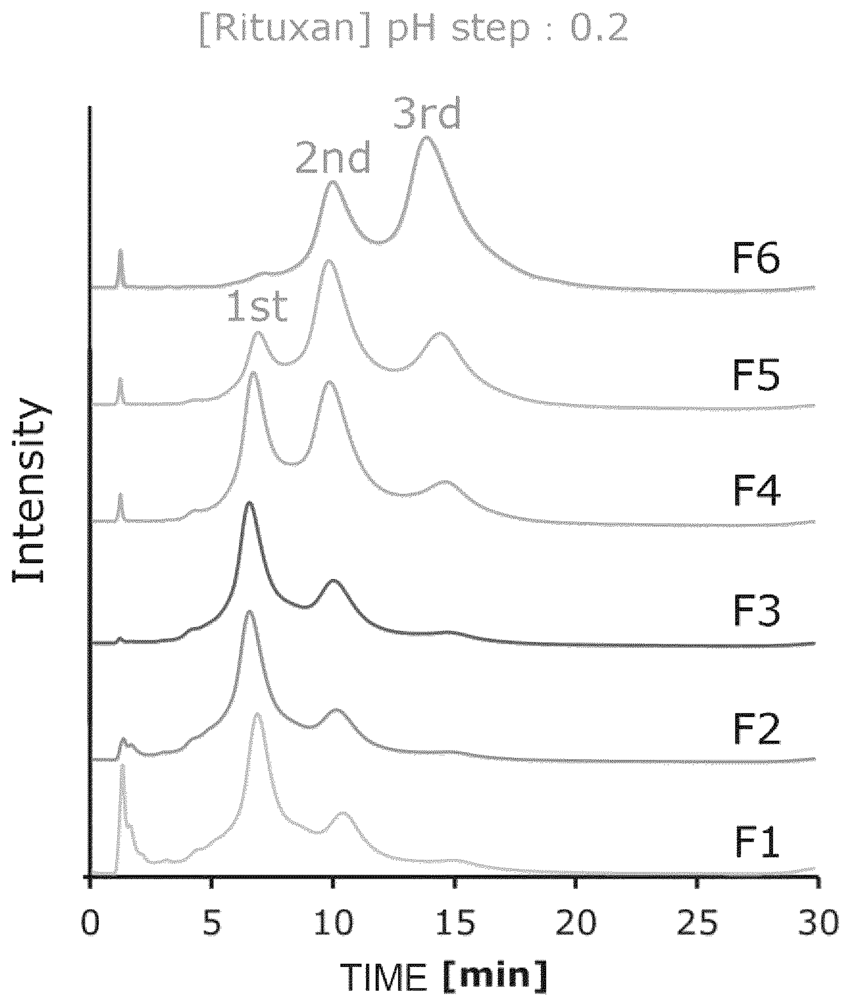
[Rituxan] pH step : 0.2
3rd
2nd
1st
F6
F5
F4
F3
F2
F1
Intensity
0
5
10
15
20
25
30
TIME [min]

*Fig.26*

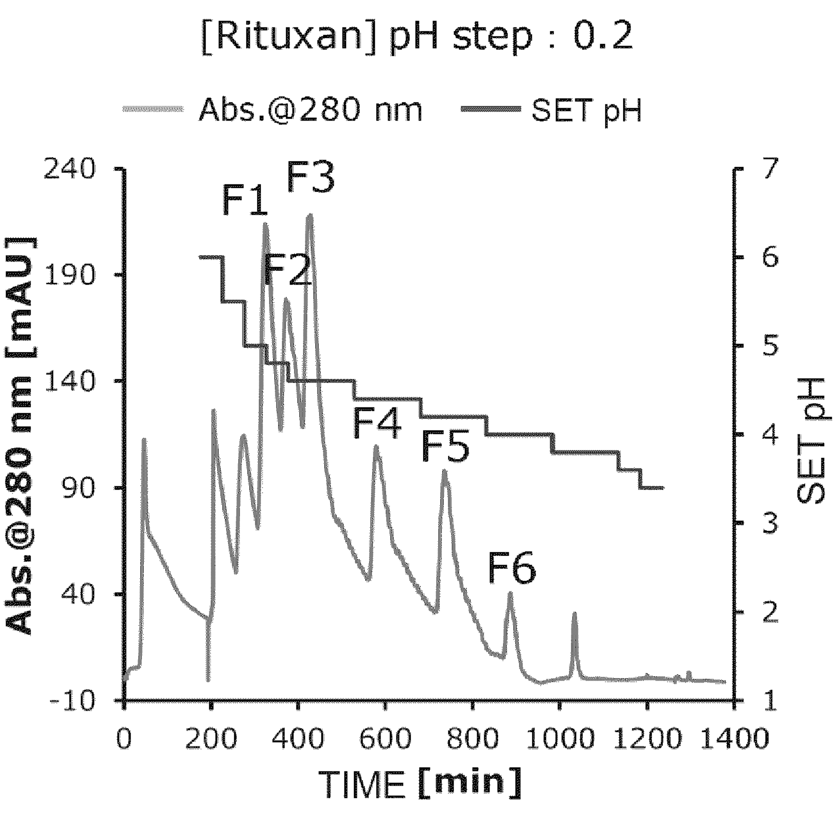
[Rituxan] pH step : 0.2
Abs.@280 nm
SET pH
240
190
140
90
40
-10
7
6
5
4
3
2
1
F1
F2
F3
F4
F5
F6
Abs.@280 nm [mAU]
SET pH
0
200
400
600
800
1000
1200
1400
TIME [min]

# Fig.27

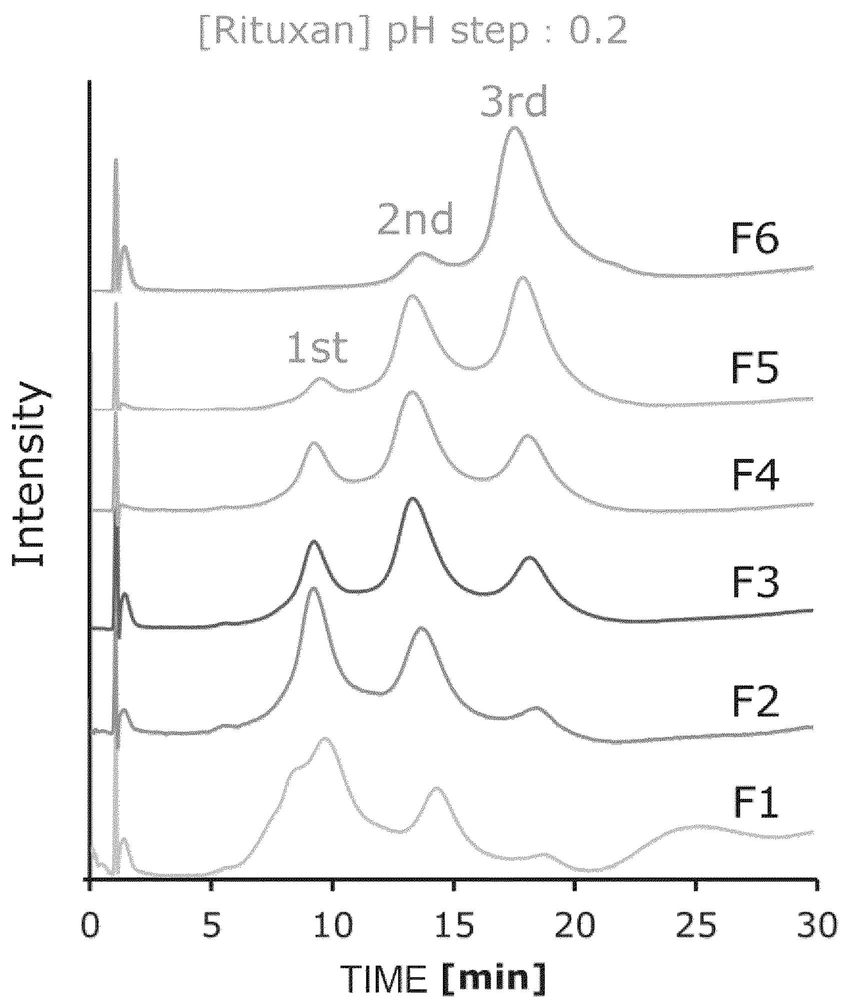
[Rituxan] pH step : 0.2
3rd
2nd
1st
F6
F5
F4
F3
F2
F1
Intensity
0
5
10
15
20
25
30
TIME [min]

*Fig.28*

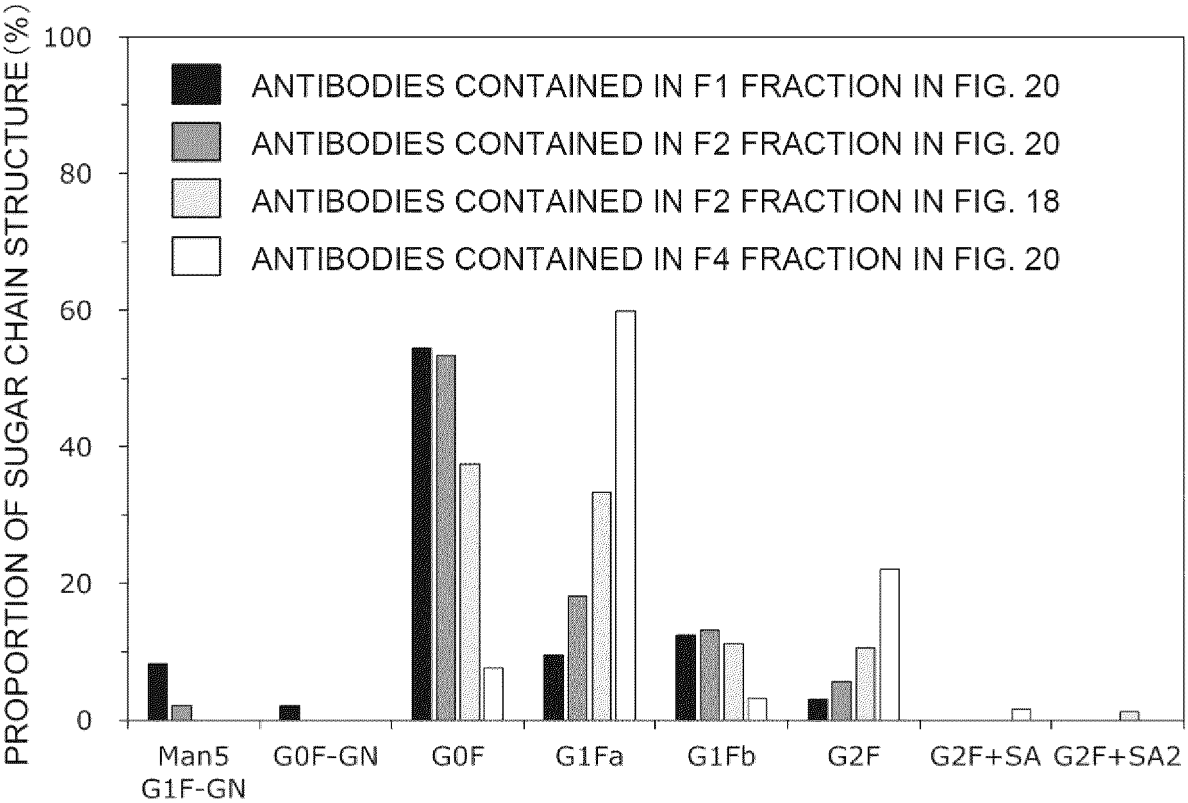
PROPORTION OF SUGAR CHAIN STRUCTURE (%)
100
80
60
40
20
0
ANTIBODIES CONTAINED IN F1 FRACTION IN FIG. 20
ANTIBODIES CONTAINED IN F2 FRACTION IN FIG. 20
ANTIBODIES CONTAINED IN F2 FRACTION IN FIG. 18
ANTIBODIES CONTAINED IN F4 FRACTION IN FIG. 20
Man5 G1F-GN
G0F-GN
G0F
G1Fa
G1Fb
G2F
G2F+SA
G2F+SA2

## INTERNATIONAL SEARCH REPORT

International application No. **PCT/JP2022/042273**

**A. CLASSIFICATION OF SUBJECT MATTER**

***C12N 15/12***(2006.01)i; ***B01J 20/281***(2006.01)i; ***C07K 1/22***(2006.01)i; ***C07K 14/735***(2006.01)i; ***C07K 16/00***(2006.01)i; ***C07K 17/02***(2006.01)i; ***G01N 30/26***(2006.01)i; ***G01N 30/34***(2006.01)i; ***G01N 30/88***(2006.01)i
FI: C12N15/12 ZNA; C07K14/735; G01N30/26 A; G01N30/34 E; G01N30/88 J; B01J20/281 G; C07K17/02; B01J20/281 R; C07K1/22; C07K16/00

According to International Patent Classification (IPC) or to both national classification and IPC

**B. FIELDS SEARCHED**

Minimum documentation searched (classification system followed by classification symbols)

C12N15/00-15/90; C07K1/00-19/00; B01J20/00-20/34; G01N30/00-30/96

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2019/083048 A1 (TOSOH CORP.) 02 May 2019 (2019-05-02) claims, paragraphs [0050], [0051], [0057], [0058], examples | 1-16 |
| A | JP 2011-132249 A (CENTELION SAS) 07 July 2011 (2011-07-07) paragraph [0161] | 1-16 |
| A | JP 2017-521389 A (LUPIN LTD.) 03 August 2017 (2017-08-03) claims, examples | 1-16 |
| A | WO 2021/210662 A1 (ONO PHARMACEUTICAL CO., LTD.) 21 October 2021 (2021-10-21) claims, examples | 1-16 |
| A | JP 2020-117454 A (KANEKA CORP.) 06 August 2020 (2020-08-06) claims, examples | 1-16 |
| A | JP 2015-511637 A (GE HEALTHCARE BIO-SCIENCES AB) 20 April 2015 (2015-04-20) claims, examples | 1-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 November 2022** | **17 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/042273**

**C. DOCUMENTS CONSIDERED TO BE RELEVANT**

| Category* | Citation of document, with indication, where appropriate, of the relevant passages | Relevant to claim No. |
|---|---|---|
| A | JP 2015-086216 A (TOSOH CORP.) 07 May 2015 (2015-05-07)<br>claims | 1-16 |

Form PCT/ISA/210 (second sheet) (January 2015)

**INTERNATIONAL SEARCH REPORT**

International application No.
**PCT/JP2022/042273**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

"Annex C/ST.25 text file format" should be read as "ST.26 format".

Form PCT/ISA/210 (continuation of first sheet) (January 2015)

**INTERNATIONAL SEARCH REPORT**
**Information on patent family members**

International application No.
**PCT/JP2022/042273**

| Patent document cited in search report | Publication date (day/month/year) | Patent family member(s) | Publication date (day/month/year) |
|---|---|---|---|
| WO 2019/083048 A1 | 02 May 2019 | US 2021/0261605 A1<br>claims, paragraphs [0124], [0125], [0131], [0132], examples<br>EP 3702462 A1<br>CN 111263813 A | |
| JP 2011-132249 A | 07 July 2011 | US 2007/0111221 A1<br>paragraph [0162]<br>CN 101006170 A<br>KR 10-2006-0135062 A | |
| JP 2017-521389 A | 03 August 2017 | US 2017/0152298 A1<br>claims, examples<br>EP 3155009 A1<br>CN 106536565 A | |
| WO 2021/210662 A1 | 21 October 2021 | (Family: none) | |
| JP 2020-117454 A | 06 August 2020 | (Family: none) | |
| JP 2015-511637 A | 20 April 2015 | US 2016/0067351 A1<br>claims, examples<br>CN 105143271 A<br>KR 10-2015-0115000 A | |
| JP 2015-086216 A | 07 May 2015 | US 2016/0222081 A1<br>claims<br>EP 3048112 A1<br>CN 105555801 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018197224 A
- WO 2017141910 A
- JP 2015086216 A
- JP 2016169197 A
- JP 2017118871 A
- WO 2019083048 A


### Non-patent literature cited in the description


- **Y. YAGI ; S. SUZUKI.** Characterization of Oligosaccharides in Therapeutic Antibodies. *CHROMATOGRAPHY,* 2013, vol. 34 (2), 83-88